(19) Europäisches Patentamt
European Patent Office
Office européen des brevets

(11) EP 2 558 092 B1

(12) **EUROPEAN PATENT SPECIFICATION**

(45) Date of publication and mention of the grant of the patent:
**27.06.2018 Bulletin 2018/26**

(21) Application number: **11715370.0**

(22) Date of filing: **12.04.2011**

(51) Int Cl.:
***A61K 31/436*** *(2006.01)* ***A61K 31/519*** *(2006.01)*
***A61K 45/06*** *(2006.01)* ***A61P 35/00*** *(2006.01)*

(86) International application number:
**PCT/US2011/032062**

(87) International publication number:
**WO 2011/130232 (20.10.2011 Gazette 2011/42)**

(54) **COMBINATION COMPRISING A CYCLIN DEPENDENT KINASE 4 OR CYCLIN DEPENDENT KINASE 6 (CDK4/6) INHIBITOR AND AN MTOR INHIBITOR FOR TREATING CANCER**

KOMBINATION MIT EINEM HEMMER DER CYCLIN-ABHÄNGIGEN KINASE-4 ODER CYCLINABHÄNGIGEN KINASE-6 (CDK4/6) UND EVEROLIMUS ZUR BEHANDLUNG VON KREBS

COMBINAISON COMPRENANT UN INHIBITEUR DE KINASE DÉPENDANTE DE CYCLINE 4 OU DE KINASE DEPENDANTE DE CYCLINE 6 (CDK4/6) ET DE L'EVEROLIMUS POUR TRAITER LE CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2010 US 323541 P**

(43) Date of publication of application:
**20.02.2013 Bulletin 2013/08**

(73) Proprietor: **Novartis AG**
**4056 Basel (CH)**

(72) Inventors:
- **BORLAND, Maria**
  **Waltham, Massachusetts 02451 (US)**
- **BRAIN, Christopher Thomas**
  **Cambridge, Massachusetts 02139 (US)**
- **DOSHI, Shivang**
  **Cambridge, Massachusetts 02139 (US)**
- **KIM, Sunkyu**
  **Cambridge, Massachusetts 02139 (US)**
- **MA, Jianguo**
  **Newton, Massachusetts 02464 (US)**
- **MURTIE, Josh**
  **Cambridge, Massachusetts 02142 (US)**
- **ZHANG, Hong**
  **Cambridge, Massachusetts 02141 (US)**

(74) Representative: **Lardans, Vinca Raymonde**
**Novartis Pharma AG**
**Patent Department**
**4002 Basel (CH)**

(56) References cited:
**WO-A1-2010/020675**
**WO-A2-2007/140222**
**WO-A2-2009/061345**
**FR-A1- 2 945 747**
**US-A1- 2005 222 163**

- **LIU Q ET AL: "MTOR mediated anti-cancer drug discovery", DRUG DISCOVERY TODAY: THERAPEUTIC STRATEGIES 2009 ELSEVIER LTD GBR LNKD- DOI:10.1016/J.DDSTR.2009.12.001, vol. 6, no. 2, July 2009 (2009-07), pages 47-55, XP0027103036, ISSN: 1740-6773**
- **GOY A: "Mantle cell lymphoma : Evolving novel options", CURRENT ONCOLOGY REPORTS, CURRENT SCIENCE, GB, vol. 9, no. 5, 1 September 2007 (2007-09-01), pages 391-398, XP008085785, ISSN: 1523-3790, DOI: DOI:10.1007/S11912-007-0053-9**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 558 092 B1

## Description

### FIELD OF THE INVENTION

A combination of a mammalian target of rapamycin (mTOR) inhibitor and a cyclin dependent kinase 4/6 (CDK4/6) inhibitor for the treatment of solid tumors and hematological malignancies. This invention also relates to the use of the combination thereof, in the management of hyperproliferative diseases like cancer.

### RELATED BACKGROUND ART

Tumor development is closely associated with genetic alteration and deregulation of CDKs and their regulators, suggesting that inhibitors of CDKs may be useful anticancer therapeutics. Indeed, early results suggest that transformed and normal cells differ in their requirement for, *e.g.,* cyclin D/CDK4/6 and that it may be possible to develop novel antineoplastic agents devoid of the general host toxicity observed with conventional cytotoxic and cytostatic drugs.

The function of CDKs is to phosphorylate and thus activate or deactivate certain proteins, including e.g. retinoblastoma proteins, lamins, histone H1, and components of the mitotic spindle. The catalytic step mediated by CDKs involves a phospho-transfer reaction from ATP to the macromolecular enzyme substrate. Several groups of compounds (reviewed in e.g. Fischer, P. M. Curr. Opin. Drug Discovery Dev. 2001, 4, 623-634) have been found to possess anti-proliferative properties by virtue of CDK-specific ATP antagonism.

At a molecular level mediation of CDK/cyclin complex activity requires a series of stimulatory and inhibitory phosphorylation, or dephosphorylation, events. CDK phosphorylation is performed by a group of CDK activating kinases (CAKs) and/or kinases such as wee1, Myt1 and Mik1. Dephosphorylation is performed by phosphatases such as cdc25(a & c), pp2a, or KAP.

CDK/cyclin complex activity may be further regulated by two families of endogenous cellular proteinaceous inhibitors: the Kip/Cip family, or the INK family. The INK proteins specifically bind CDK4 and CDK6. $p16^{ink4}$ (also known as MTS1) is a potential tumour suppressor gene that is mutated, or deleted, in a large number of primary cancers. The Kip/Cip family contains proteins such as $p21^{Cip1,Waf1}$, $p27^{Kip1}$ and $p57^{kip2}$, where p21 is induced by p53 and is able to inactivate the CDK2/cyclin(E/A) complex. Atypically low levels of p27 expression have been observed in breast, colon and prostate cancers. Conversely over expression of cyclin E in solid tumours has been shown to correlate with poor patient prognosis. Over expression of cyclin D1 has been associated with oesophageal, breast, squamous, and non-small cell lung carcinomas.

The pivotal roles of CDKs, and their associated proteins, in co-ordinating and driving the cell cycle in proliferating cells have been outlined above. Some of the biochemical pathways in which CDKs play a key role have also been described. The development of monotherapies for the treatment of proliferative disorders, such as cancers, using therapeutics targeted generically at CDKs, or at specific CDKs, is therefore potentially highly desirable. Thus, there is a continued need to find new therapeutic agents to treat human diseases.

mTOR is a kinase protein predominantly found in the cytoplasm of the cell. It acts as a central regulator of many biological processes related to cell proliferation, angiogenesis, and cell metabolism. mTOR exerts its effects primarily by turning on and off the cell's translational machinery, which includes the ribosomes, and is responsible for protein synthesis. mTOR is a key intracellular point of convergence for a number of cellular signaling pathways. mTOR performs its regulatory function in response to activating or inhibitory signals transmitted through these pathways, which are located upstream from mTOR in the cell. These diverse signaling pathways are activated by a variety of growth factors (including vascular endothelial growth factors (VEGFs), platelet-derived growth factor (PDGF), epidermal growth factor (EGF), insulin-like growth factor 1 (IGF-1)), hormones (estrogen, progesterone), and the presence or absence of nutrients (glucose, amino acids) or oxygen. One or more of these signaling pathways may be abnormally activated in patients with many different types of cancer, resulting in deregulated cell proliferation, tumor angiogenesis, and abnormal cell metabolism.

US 2005/222163 discloses a method of treating abnormal cell growth, in particular cancer, using a combination of a CDK/inhibitor and an mTor inhibitor. Liu Q. et al, Drug Discovery Today: Therapeutic Strategies 2009, Vol. 6, no. 2, 47-55 is a review article that focuses on the development of small molecule ATP-competitive inhibitors of mTOR and their potential therapeutic uses. Goy A, Current Oncology Reports 2007, vol. 9, no. 5, 391-398 disusses evolving approaches to the treatment of mantle cell lymphoma. These include mTor and Bcl-2 inhibitors, novel antibodies and new cytotoxic agents.

### BRIEF SUMMARY OF THE INVENTION

The invention provides a combination comprising a first agent that inhibits the CDK4/6 pathway and a second agent that inhibits mTOR, i.e. the kinase activity of mTOR and its downstream effectors as described in the claims.

The invention provides a combination comprising a first agent that is a cyclin dependent kinase 4 or cyclin dependent kinase 6 (CDK4/6) inhibitor, and a second agent that is an mTOR inhibitor, wherein the first agent is 7-Cyclopentyl-2-(5-piperazin-1-yl-pyridin-2-ylamino)-7H-pyrrolo[2,3-d]pyrimidine-6-carboxylic acid dimethylamide, or a pharmaceutically acceptable salt, and the second agent is everolimus. The invention also provides this combination for use in the treatment of cancer and this combination for use in the treatment of cancer as described in the claims.

In another aspect, the invention provides combinations including pharmaceutical compositions comprising a therapeutically effective amount of a first agent that inhibits CDK4/6, a second agent that inhibits the kinase activity of mTOR and downstream effectors, and a pharmaceutically acceptable carrier, wherein the first agent is 7-Cyclopentyl-2-(5-piperazin-1-yl-pyridin-2-ylamino)-7H-pyrrolo[2,3-d]pyrimidine-6-carboxylic acid dimethylamide, or a pharmaceutically acceptable salt, and the second agent is everolimus.

Furthermore, the present invention provides for the use of a therapeutically effective amount of a combination comprising a first agent that inhibits the CDK4/6 pathway and a second agent that inhibits the kinase activity of mTOR and downstream effectors, or a pharmaceutically acceptable salt or pharmaceutical composition thereof, in the manufacture of a medicament for treating cancer, wherein the first agent is 7-Cyclopentyl-2-(5-piperazin-1-yl-pyridin-2-ylamino)-7H-pyrrolo[2,3-d]pyrimidine-6-carboxylic acid dimethylamide, or a pharmaceutically acceptable salt and the second agent is everolimus.

The present invention has a therapeutic use in the treatment of cancer, particularly retinoblastoma protein (retinoblastoma tumor suppressor protein or pRb) positive cancers. Types of such cancers include mantle cell lymphoma, pancreatic cancer, breast cancer, non small cell lung cancer, melanoma, colon cancer, esophageal cancer and liposarcoma.

The above combinations and compositions can be administered to a system comprising cells or tissues, as well as a human patient or and animal subject.

## BRIEF DESCRIPTION OF THE DRAWINGS

Figure 1 shows enhanced growth inhibitions by CDK4/6 and mTOR inhibitor combinations. Jeko-1 mantle cell lymphoma cells were used to evaluate the effects on cell growth. % growth compared to control (100%) is shown. Compound A1 is a CDK4/6 inhibitor and Compound B1 is an mTOR inhibitor. A1+B1 combinations are growth inhibitions observed when Jeko-1 cells were co-treated with A1 and B1 compounds at the same time. Actual concentrations used are shown in the graphs.

Figure 2 is an isobologram analysis of a CDK4/6 and mTOR inhibitor combination in a Jeko-1 mantle cell lymphoma cell line. Compound A1 and B1 are CDK4/6 and mTOR inhibitors, respectively. The graph shown was constructed using the concentrations that gave 50% growth inhibitions. Dotted Line 1 represents the growth inhibitions predicted for a simple additivity when the effects of A1 and B1 are combined. Line 2 is the observed growth inhibitions, indicating that A1/B1 combination results in strong synergistic growth inhibition.

Figure 3 is an isobologram analysis of a CDK4/6 and mTOR inhibitor combination in a MDA-MB453 breast cancer cell line. Compound A1 and B1 are CDK4/6 and mTOR inhibitors, respectively. Similar to Figure 2 above, the graph shown was constructed using the concentrations that gave 50% growth inhibitions, with dotted Line 1 representing the growth inhibitions predicted for a simple additivity. Line 2 is the observed growth inhibitions, indicating that A1/B1 combination results in strong synergistic growth inhibition.

Figure 4 shows that a combination of Compound A1 with Compound B1 enhanced tumor growth delay in the Jeko-1 mantle cell lymphoma xenograft model. Dosing was stopped 35 days post treatment initiation (56 days post implantation) and tumors were allowed to re-grow. The combination dosing group had significantly enhanced tumor growth delay (20 days).

Figure 5 is a combination of Compound A1 with Compound B1 that enhanced tumor growth delay and tumor growth inhibition in the PANC-1 pancreatic carcinoma xenograft model, for tumor volume (Fig. 5A) and percentage alive (Fig. 5B). Dosing was stopped 22 days post treatment initiation and tumors were allowed to re-grow. The combination dosing group had significantly enhanced tumor growth delay (18 days).

Figure 6 illustrates, when the combination of CDK4/6 inhibitor Compound A1 and mTOR inhibitor Compound B1, is used to treat Jeko-1 cells, the resulting inhibition values were used by CHALICE software to generate Inhibition and ADD Excess Inhibition matrices, as well as the isobolograms.

Figure 7 illustrates, when the combination of CDK4/6 inhibitor Compound A1 and mTOR inhibitor Compound B2, is used to treat Jeko-1 cells, the resulting inhibition values were used by CHALICE software to generate Inhibition and ADD Excess Inhibition matrices, as well as the isobolograms.

Figure 8 illustrates, when the combination of CDK4/6 inhibitor Compound A4 and mTOR inhibitor Compound B1, is used to treat Jeko-1 cells, the resulting inhibition values were used by CHALICE software to generate Inhibition

and ADD Excess Inhibition matrices, as well as the isobolograms.

Figure 9 illustrates, when the combination of CDK4/6 inhibitor Compound A2 and mTOR inhibitor Compound B1, is used to treat Jeko-1 cells, the resulting inhibition values were used by CHALICE software to generate Inhibition and ADD Excess Inhibition matrices, as well as the isobolograms.

Figure 10 illustrates, when the combination of CDK4/6 inhibitor Compound A3 and mTOR inhibitor Compound B1, is used to treat Jeko-1 cells, the resulting inhibition values were used by CHALICE software to generate Inhibition and ADD Excess Inhibition matrices, as well as the isobolograms.

Figure 11 illustrates, when the combination of CDK4/6 inhibitor Compound A6 and mTOR inhibitor Compound B1, is used to treat Jeko-1 cells, the resulting inhibition values were used by CHALICE software to generate Inhibition and ADD Excess Inhibition matrices, as well as the isobolograms.

Figure 12 illustrates, when the combination of CDK4/6 inhibitor Compound A5 and mTOR inhibitor Compound B1, is used to treat Jeko-1 cells, the resulting inhibition values were used by CHALICE software to generate Inhibition and ADD Excess Inhibition matrices, as well as the isobolograms.

Figure 13 illustrates, when the combination of CDK4/6 inhibitor Compound A4 and mTOR inhibitor Compound B2, is used to treat Jeko-1 cells, the resulting inhibition values were used by CHALICE software to generate Inhibition and ADD Excess Inhibition matrices, as well as the isobolograms.

Figure 14 illustrates, when the combination of CDK4/6 inhibitor Compound A2 and mTOR inhibitor Compound B2, is used to treat Jeko-1 cells, the resulting inhibition values were used by CHALICE software to generate Inhibition and ADD Excess Inhibition matrices, as well as the isobolograms.

Figure 15 illustrates, when the combination of CDK4/6 inhibitor Compound A3 and mTOR inhibitor Compound B2, is used to treat Jeko-1 cells, the resulting inhibition values were used by CHALICE software to generate Inhibition and ADD Excess Inhibition matrices, as well as the isobolograms.

Figure 16 illustrates, when the combination of CDK4/6 inhibitor Compound A6 and mTOR inhibitor Compound B2, is used to treat Jeko-1 cells, the resulting inhibition values were used by CHALICE software to generate Inhibition and ADD Excess Inhibition matrices, as well as the isobolograms.

Figure 17 illustrates, when the combination of CDK4/6 inhibitor Compound A5 and mTOR inhibitor Compound B2, is used to treat Jeko-1 cells, the resulting inhibition values were used by CHALICE software to generate Inhibition and ADD Excess Inhibition matrices, as well as the isobolograms.

## DETAILED DESCRIPTION OF THE INVENTION

Mammalian cell cycle progression is a tightly controlled process in which transitions through different phases are conducted in a highly ordered manner and guarded by multiple checkpoints. The retinoblastoma protein (pRb) is the checkpoint protein for G1 to S phase transition, which associates with a family of E2F transcription factors to prevent their activity in the absence of appropriate growth stimuli. Upon mitogen stimulation, quiescent cells begin their entry into S phase by newly synthesizing D-cyclins, which are the activators of cyclin dependent kinases 4 and 6 (CDK4/6). Once bound by the cyclins, CDK4/6 deactivate the pRb protein via phosphorylation and this releases E2F to direct transcription of genes required for S phase. Full deactivation of pRb requires phosphorylations by both cyclin D-CDK4/6 and cyclin E-CDK2, where phosphorylations by CDK4/6 at specific sites of pRb (Ser780, Ser795) have been shown to be a prerequisite for cyclin E-CDK2 phosphorylation. In addition to D-cyclins, the activity of CDK4/6 is regulated by p16, encoded by INK4a gene, which inhibits the kinase activity. The CIP/KIP proteins, which are the inhibitors of cyclin E-CDK2, also bind to cyclin D-CDK4/6 complex, and this results in further activation of CDK2 by sequestering the CIP/KIP away from their target. Therefore, the cyclin D-CDK4/6 is a key enzyme complex that regulates the G1 to S phase transition.

The D-cyclin-CDK4/6-INK4a-pRb pathway is universally disrupted to favor cell proliferation in cancer. In a majority of cases (~80%), cancers maintain a functional pRb and utilize different mechanisms to increase the CDK4/6 kinase activity. One of the most common events is the inactivation of p16 via mutations, deletions and epigenetic silencing. Indeed, the functional absence of p16 is frequently observed in large portions of non small cell lung cancer, melanoma, pancreatic cancer and mesothelioma. Coupled with the observation that a specific mutation of the CDK4 gene (CDKR24C), that confers resistance to p16 binding, has been shown to play a causal role in a familial melanoma, the growth advantage provided by unchecked CDK4/6 activity appear to be one of the key elements associated with a tumor development.

Another mechanism to enhance the kinase activity is to increase the abundance of D-cyclins and this is accomplished by translocation, amplification and overexpression of the gene. Cyclin D1 gene is translocated to the immunoglobulin heavy chain in a majority of mantle cell lymphoma and this aberration leads to constitutive expression of the gene resulting in unchecked cell proliferation. The translocation is also observed in many cases of multiple myeloma. The example of the gene amplification is seen in squamous cell esophageal cancer, where approximately 50% of the cases have been reported to harbor cyclin D1 amplifications. This suggests that a large portion of the esophageal cancer

may be highly dependent on activated kinases for growth. Cyclin D1 amplification is also often detected in breast cancers. In addition to the genetic defects directed related to the cyclin D1 gene, its transcription can also be profoundly elevated by activated oncogenes that are upstream regulators of the gene. Activated Ras or Neu oncogenes have been shown to promote breast cancer in mice by primarily upregulating cyclin D1. Suppression of the cyclin D1 levels or inhibition of the kinase activity were able to prevent tumor growth in both initiation and maintenance phases, demonstrating that an unchecked CDK4/6 was the key element in the development of the cancers. Other activating aberrations of mitogen pathways such as V600E B-Raf in MAPK and PTEN deletions in PI3K also increase D-cyclins to achieve accelerated proliferations, suggesting CDK4/6 may also be crucial for the cancers bearing the. Lastly, the genes encoding CDK4 and 6 are also amplified in subset of human neoplasms. CDK4 gene is amplified in 100% of liposarcomas along with MDM2 gene, while CDK6 is frequently amplified in T-LBL/ALL. Taken together, CDK4/6 appears to be a crucial protein necessary for proliferation of numerous human cancers with a functional pRb, including mantle cell lymphoma, pancreatic cancer, breast cancer, non small cell lung cancer, melanoma, colon cancer, esophageal cancer and liposarcoma.

Disclosed herein is a combination comprising a first agent that is a cyclin dependent kinase 4/6(CDK4/6) inhibitor and a second agent that is an mTOR inhibitor, wherein the first agent is a compound of Formula I:

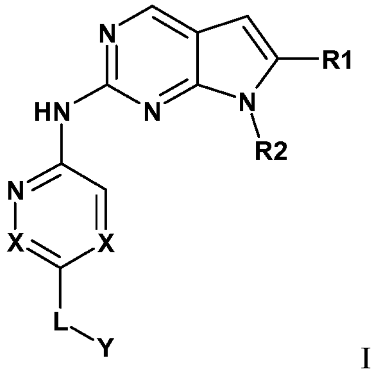

I

or a pharmaceutically acceptable salt thereof, wherein

X is $CR^9$, or N;
$R^1$ is $C_{1-8}$alkyl, CN, C(O)$OR^4$ or $CONR^5R^6$, a 5-14 membered heteroaryl group, or a 3-14 membered cycloheteroalkyl group;
$R^2$ is $C_{1-8}$alkyl, $C_{3-14}$cycloalkyl, or a 5-14 membered heteroaryl group, and wherein $R^2$ may be substituted with one or more $C_{1-8}$alkyl, or OH;
L is a bond, $C_{1-8}$alkylene, C(O), or C(O)$NR^{10}$, and wherein L may be substituted or unsubstituted;
Y is H, $R^{11}$, $NR^{12}R^{13}$, OH, or Y is part of the following group

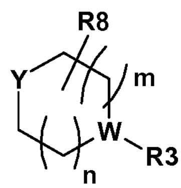

,

where Y is $CR^9$ or N;
where 0-3 $R^8$may be present, and $R^8$ is $C_{1-8}$alkyl, oxo, halogen, or two or more $R^8$ may form a bridged alkyl group;
W is $CR^9$, or N;
$R^3$ is H, $C_{1-8}$alkyl, $C_{1-8}$alkyl$R^{14}$, $C_{3-14}$cycloalkyl, C(O)$C_{1-8}$ alkyl, $C_{1-8}$haloalkyl, $C_{1-8}$alkylOH, C(O)$NR^{14}R^{15}$, $C_{1-8}$cyanoalkyl, C(O)$R^{14}$, $C_{0-8}$alkylC(O)$C_{0-8}$alkyl$NR^{14}R^{15}$, $C_{0-8}$alkylC(O)$OR^{14}$, $NR^{14}R^{15}$, $SO_2C_{1-8}$alkyl, $C_{1-8}$alkyl$C_{3-14}$cycloalkyl, C(O)$C_{1-8}$alkyl$C_{3-14}$cycloalkyl, $C_{1-8}$alkoxy, or OH which may be substituted or unsubstituted when $R^3$ is not H.
$R^9$ is H or halogen;
$R^4$, $R^5$, $R^6$, $R^7$ , $R^{10}$, $R^{11}$ , $R^{12}$, $R^{13}$, $R^{14}$, and $R^{15}$ are each independently selected from H, $C_{1-8}$alkyl, $C_{3-14}$ cycloalkyl, a 3-14 membered cycloheteroalkyl group, a $C_{6-14}$ aryl group, a 5-14 membered heteroaryl group, alkoxy, C(O)H, C(N)OH, C(N)$OCH_3$, C(O)$C_{1-3}$alkyl, $C_{1-8}$alkyl$NH_2$, $C_{1-6}$alkylOH, and wherein $R^4$, $R^5$, $R^6$, $R^7$, $R^{10}$, $R^{11}$ , $R^{12}$, and $R^{13}$, $R^{14}$, and $R^{15}$ when not H may be substituted or unsubstituted;

m and n are independently 0-2; and
wherein L, $R^3$, $R^4$, $R^5$ , $R^6$, $R^7$, $R^{10}$, $R^{11}$, $R^{12}$, and $R^{13}$, $R^{14}$, and $R^{15}$ may be substituted with one or more of $C_{1-8}$alkyl, $C_{2-8}$alkenyl, $C_{2-8}$alkynyl, $C_{3-14}$cycloalkyl, 5-14 membered heteroaryl group, $C_{6-14}$aryl group, a 3-14 membered cycloheteroalkyl group, OH, (O), CN, alkoxy, halogen, or $NH_2$.

In an embodiment of the first general embodiment, the combination includes a CDK4/6 inhibitor of Formula I, wherein $R^3$ is H, $C_{1-8}$alkyl, $C_{3-14}$cycloalkyl, C(O)$C_{1-8}$ alkyl, $C_{1-8}$alkylOH, $C_{1-8}$cyanoalkyl, $C_{0-8}$alkylC(O)$C_{0-8}$alkylN$R^{14}R^{15}$, $C_{0-8}$alkylC(O)O$R^{14}$, N$R^{14}R^{15}$, $C_{1-8}$alkyl$C_{3-14}$cycloalkyl, C(O)$C_{1-8}$alkyl$C_{3-14}$cycloalkyl, $C_{0-8}$alkoxy, $C_{1-8}$alkyl$R^{14}$, $C_{1-8}$haloalkyl, or C(O)$R^{14}$, which may be substituted with one or more of OH, CN, F, or $NH_2$, and wherein $R^{14}$ and $R^{15}$ are each independently selected from H, $C_{1-8}$alkyl, $C_{3-14}$cycloalkyl, alkoxy, C(O)$C_{1-3}$alkyl, $C_{1-8}$alkyl$NH_2$, or $C_{1-6}$alkylOH.

The compound of Formula I present in the combination of the present invention is
7-Cyclopentyl-2-(5-piperazin-1-yl-pyridin-2-ylamino)-7H-pyrrolo[2,3-d]pyrimidine-6-carboxylic acid dimethylamide
or a pharmaceutically acceptable salt thereof.

The compounds of Formula (I) are generally and specifically described in published PCT patent application WO2010/020675.

Specific exemplary cyclin dependent kinase 4/6(CDK4/6) inhibitors include, but not limited to:

Compound A1: 7-Cyclopentyl-2-(5-piperazin-1-yl-pyridin-2-ylamino)-7H-pyrrolo[2,3-d]pyrimidine-6-carboxylic acid dimethylamide, which has the following chemical structure

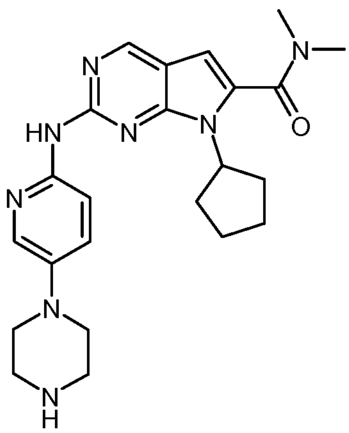

.

Compound A2: 7-Cyclopentyl-2-[5-(3,8-diaza-bicyclo[3.2.1]octane-3-carbonyl)-pyridin-2-ylamino]-7H-pyrrolo[2,3-d]pyrimidine-6-carboxylic acid dimethylamide, which has the following chemical structure:

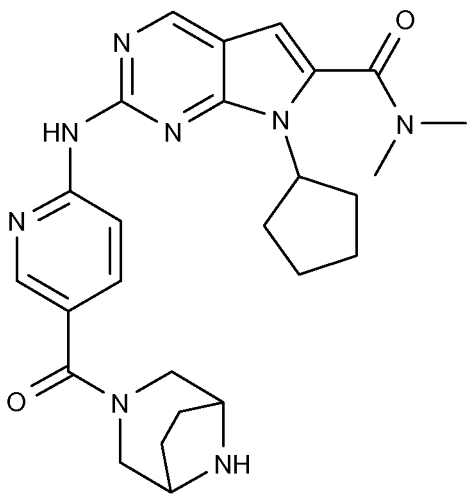

.

Compound A3: 7-Cyclopentyl-2-[5-((1R,6S)-9-methyl-4-oxo-3,9-diaza-bicyclo[4.2.1]non-3-yl)-pyridin-2-ylamino]-

7H-pyrrolo[2,3-d]pyrimidine-6-carboxylic acid dimethylamide, which has the following chemical structure:

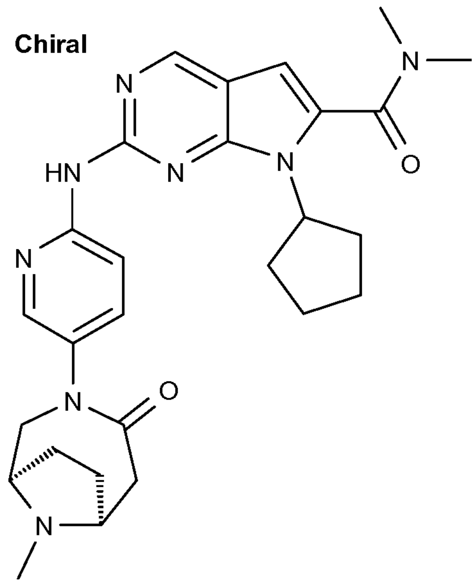

.

Compound A4: 6-Acetyl-8-cyclopentyl-5-methyl-2-(5-piperazin-1-yl-pyridin-2-ylamino)-8H-pyrido[2,3-d]pyrimidin-7-one, which has the following chemical structure:

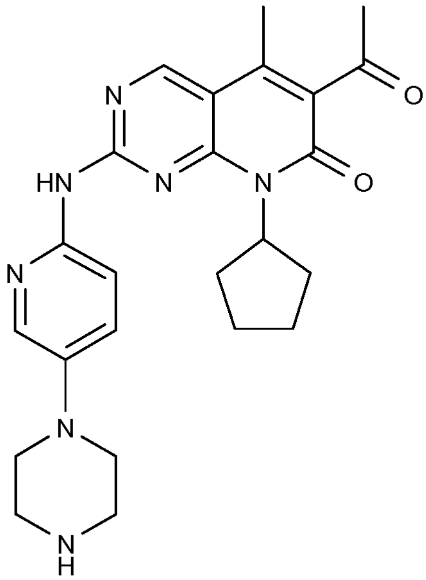

.

Compound A5: N*6'*-[4-(5-Isopropyl-3-trifluoromethyl-1H-pyrazol-4-yl)-pyrimidin-2-yl]-N*4*,N*4*-dimethyl-3,4,5,6-tetrahydro-2H-[1,3']bipyridinyl-4,6'-diamine, which has the following chemical structure:

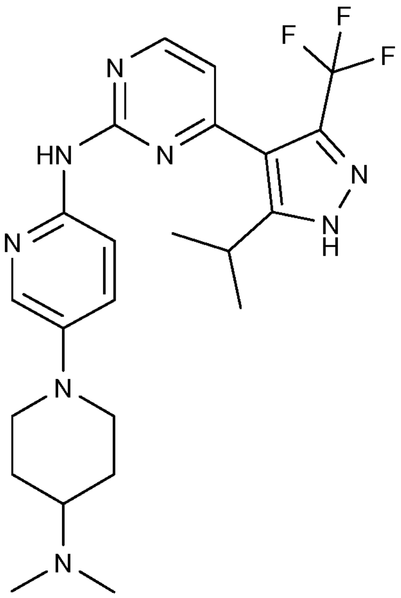

.

Compound A6: [4-(5-Isopropyl-1H-pyrazol-4-yl)-pyrimidin-2-yl]-(5-piperazin-1-yl-pyridin-2-yl)-amine, which has the following chemical structure:

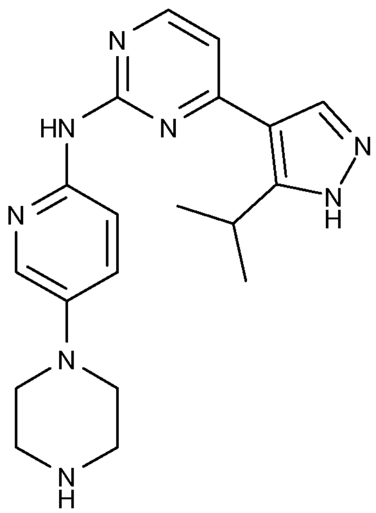

.

The mTOR inhibitor in the combination of the present invention is Everolimus (RAD001, Novartis).

Everolimus, which is Compound B1, has the chemical name((1R,9S,12S,15R,16E,18R,19R,21R,23S,24E,26E,28E,30S,32S,35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9] hexatriaconta-16,24, 26,28-tetraene-2,3,10,14,20-pentaone.) Everolimus and analogues are described in US Patent No. 5,665,772, at column 1, line 39 to column 3, line 11. Everolimus is described by the following structure:

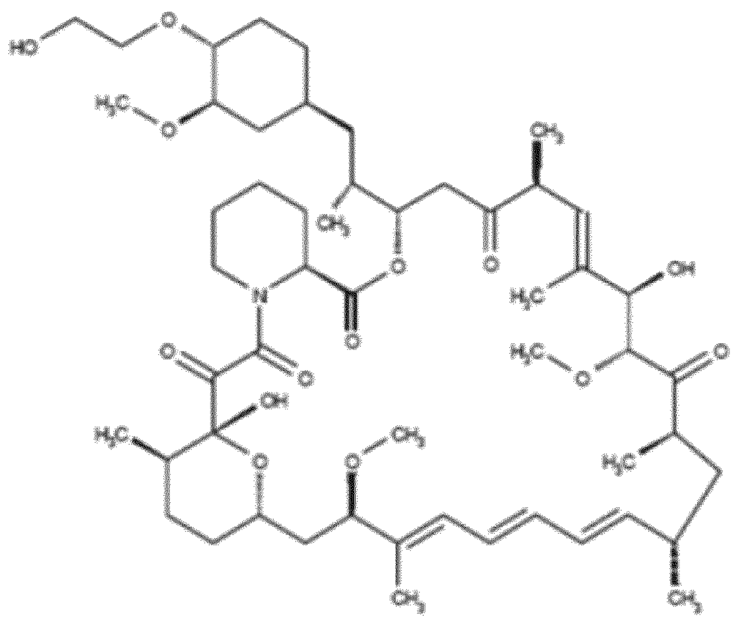

Rapamycin, which is Compound B2, is an mTOR inhibitor and has the chemical name (3S,6R,7E,9R,10R,12R,14S,15E,17E,19E,21S,23S,26R,27R,34aS)-9,10,12,13,14,21,22,23,24,25,26,27,32,33,34,34a-hexadecahydro-9,27-dihydroxy-3-[(1R)-2-[(1S,3R,4R)-4-hydroxy-3-methoxycyclohexyl]-1-methylethyl]-10,21-dimethoxy-6,8,12,14,20,26-hexamethyl-23,27-epoxy-3H-pyrido[2,1-c][1,4]-oxaazacyclohentriacontine-1,5,11,28,29(4H,6H,31H)-pentone. It is described by the following structure:

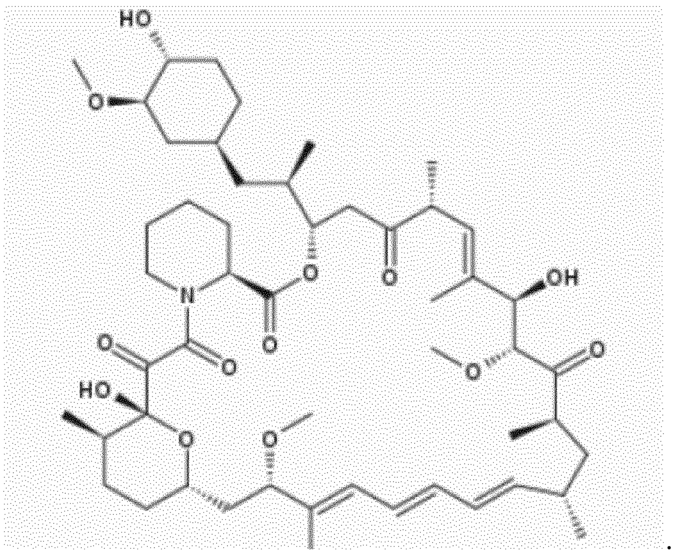

.

In another embodiment, the present invention includes a combination of the present invention for use in a method of treating a hyperproliferative disease, preferably cancer, dependent on CDK4/6 or mTOR. CDK4/6 dependent cancers are also generally marked by a hyperphosphorlyated (retinoblastoma) Rb protein. A cancer is dependent on a pathway if inhibiting or blocking that pathway will slow or disrupt growth of that cancer. Examples of CDK4 or CDK6 pathway dependent cancers include breast cancer, non small cell lung cancer, melanoma, colon cancer, esophageal cancer, liposarcoma, mantle cell lyomphoma, multiple myeloma, T-cell leukemia, renal cell carcinoma, gastric cancer and pancreatic cancer. Examples of mTOR pathway dependent cancers include breast cancer, pancreatic cancer, renal cell carcinoma, mantle cell lymphoma, glioblastoma, hepatocellular carcinoma, gastric cancer, lung cancer and colon cancer. Correlation of cancers with the CDK4/6 pathway or the mTOR pathway has been established in the art. For example, see Shapiro, Journal of Clinical Oncology, Vol. 24, No. 11 (2006) pp. 1770-1783 or Fasolo, Expert Opin. Investig. Drugs Vol. 17, No. 11 (2008) pp. 1717-1734.

Therefore in an embodiment of the invention is a combination of a CDK4/6 inhibitor and an mTOR inhibition

for use in treating cancer, by manufacture in a medicament, which can be sold as either a combined or separate dosage form, or a method of treating cancer by administering the combination to a patient in need thereof. The cancer can be a solid tumor cancer or a lymphoma. Preferred cancers include pancreatic cancer, breast cancer, mantle cell lyomphoma, non small cell lung cancer, melanoma, colon cancer, esophageal cancer, liposarcoma, multiple myeloma, T-cell leukemia, renal cell carcinoma, gastric cancer, renal cell carcinoma, glioblastoma, hepatocellular carcinoma, gastric cancer, lung cancer or colon cancer.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

The phrase "therapeutically effective amount" is used herein to mean an amount sufficient to reduce by at least about 15 percent, preferably by at least 50 percent, more preferably by at least 90 percent, and most preferably prevent, a clinically significant deficit in the activity, function and response of the host. Alternatively, a therapeutically effective amount is sufficient to cause an improvement in a clinically significant condition/symptom in the host.

"Agent" refers to all materials that may be used to prepare pharmaceutical and diagnostic compositions, or that may be compounds, nucleic acids, polypeptides, fragments, isoforms, variants, or other materials that may be used independently for such purposes, all in accordance with the present invention.

"Analog" as used herein, refers to a small organic compound, a nucleotide, a protein, or a polypeptide that possesses similar or identical activity or function(s) as the compound, nucleotide, protein or polypeptide or compound having the desired activity and therapeutic effect of the present invention. (e.g., inhibition of tumor growth), but need not necessarily comprise a sequence or structure that is similar or identical to the sequence or structure of the preferred embodiment

The present disclosure includes all pharmaceutically acceptable isotopically-labelled compounds of the invention, i.e. compounds of Formula (I), wherein one or more atoms are replaced by atoms having the same atomic number, but an atomic mass or mass number different from the atomic mass or mass number usually found in nature.

Examples of isotopes suitable for inclusion in the compounds of the disclosure comprises isotopes of hydrogen, such as $^{2}H$ and $^{3}H$, carbon, such as $^{11}C$, $^{13}C$ and $^{14}C$, chlorine, such as $^{36}Cl$, fluorine, such as $^{18}F$, iodine, such as $^{123}I$ and $^{125}I$, nitrogen, such as $^{13}N$ and $^{15}N$, oxygen, such as $^{15}O$, $^{17}O$ and $^{18}O$, phosphorus, such as $^{32}P$, and sulphur, such as $^{35}S$.

Certain isotopically-labelled compounds of Formula (I), for example, those incorporating a radioactive isotope, are useful in drug and/or substrate tissue distribution studies. The radioactive isotopes tritium, *i.e.* $^{3}H$, and carbon-14, *i.e.* $^{14}C$, are particularly useful for this purpose in view of their ease of incorporation and ready means of detection.

Substitution with heavier isotopes such as deuterium, *i.e.* $^{2}H$, may afford certain therapeutic advantages resulting from greater metabolic stability, for example, increased *in vivo* half-life or reduced dosage requirements, and hence may be preferred in some circumstances.

Substitution with positron emitting isotopes, such as $^{11}C$, $^{18}F$, $^{15}O$ and $^{13}N$, can be useful in Positron Emission Topography (PET) studies for examining substrate receptor occupancy.

Isotopically-labeled compounds of Formula (I) can generally be prepared by conventional techniques known to those skilled in the art or by processes analogous to those described in the accompanying Examples and Preparations using an appropriate isotopically-labelled reagents in place of the non-labelled reagent previously employed.

## EXAMPLES

Examples 1-3 illustrate the general procedure can be used to make 7-Cyclopentyl-2-(5-piperazin-1-yl-pyridin-2-ylamino)-7H-pyrrolo[2,3-d]pyrimidine-6-carboxylic acid dimethylamide (Compound A1). Additional methods for making the CDK4/6 inhibitors described herein can be found in WO Application No. PCT/EP09/060793, published as WO 2010/020675.

### Reference Example 1

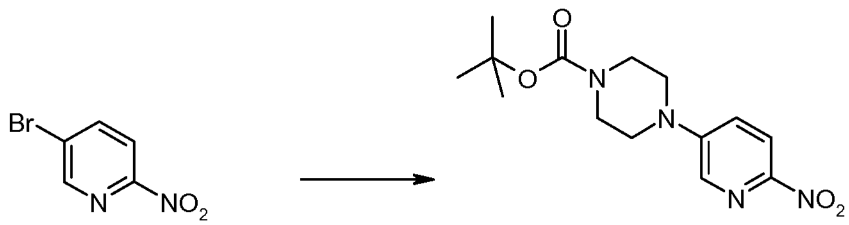

Nitrile analogues can be made by the following. To a stirred solution of 5-bromo-2-nitropyridine (4.93 g, 24.3 mmol) and piperazine-1-carboxylic acid *tert*-butyl ester (4.97 g, 26.7 mmol) in $CH_3CN$ (60 ml) is added DIPEA (4.65 mL, 26.7 mmol). The mixture is heated at reflux for 72 hours then cooled to room temperature and the precipitated product collected by filtration. The filtrate is concentrated and purified by flash column chromatography eluting with 30% EtOAc/petrol. The combined products are re-crystallized from EtOAc/petrol to give 4-(6-nitro-pyridin-3-yl)-piperazine-1-carboxylic acid *tert*-butyl ester, (4.50 g, 80% yield). MS(ESI) *m/z* 308 $(M+H)^+$

Reference Example 2

A mixture of 5-[4-(2,2,2-trifluoro-ethyl)-piperazin-1-yl]-pyridin-2-ylamine (158mg, 0.607mmol), 2-chloro-7-cyclopentyl-7H-pyrrolo[2,3-d]pyrimidine-6-carboxylic acid dimethylamide (118 mg, 0.405mmol), $Pd_2(dba)_3$ (18.5mg, 0.020mmol), BINAP (25mg, 0.040mmol) and sodium-*tert*-butoxide (70mg, 0.728mmol) in dioxane (3.5mL) is degassed and heated to 100°C for 1 h in a CEM Discover microwave. The reaction mixture is partitioned between dichloromethane and saturated $NaHCO_3$ solution. The organic layer is separated and the aqueous layer extracted with further dichloromethane. The combined organics are ished with brine, dried ($MgSO_4$), filtered and concentrated. The crude product is purified using silica gel chromatography (0 to 10% methanol/dichloromethane) to give 7-cyclopentyl-2-{5-[4-(2,2,2-trifluoro-ethyl)-piperazin-1-yl]-pyridin-2-ylamino}-7H-pyrrolo[2,3-d]pyrimidine-6-carboxylic acid dimethylamide, which is purified further by trituration with acetonitrile (115mg, 55%). MS(ESI) *m/z* 517.2 $(M+H)^+$ (method A).
$^1H$ NMR (400 MHz, Me-$d_3$-OD): 8.72 (1H, s), 8.24 (1H, d), 7.98 (1H, d), 7.50 (1H, dd), 6.62 (1H, s), 4.81-4.72 (1H, m), 3.27-3.09 (12H, m), 2.89 (4H, t), 2.61-2.49 (2H, m), 2.16-2.01 (4H, m), 1.81-1.69 (2H, m).

Reference Example 3

7-Cyclopentyl-2-(5-piperazin-1-yl-pyridin-2-ylamino)-7H-pyrrolo[2,3-d]pyrimidine-6-carboxylic acid dimethylamide

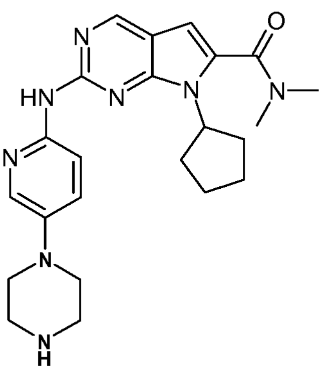

Following Buchwald Method of Example 2, then General Procedure of Example 1, 2-chloro-7-cyclopentyl-7H-pyrrolo[2,3-d]pyrimidine-6-carboxylic acid dimethylamide (300 mg, 1.02 mmol) and 5-piperazin-1-yl-pyridin-2-ylamine (314 mg, 1.13 mmol) gave 7-cyclopentyl-2-(5-piperazin-1-yl-pyridin-2-ylamino)-7H-pyrrolo[2,3-d]pyrimidine-6-carboxylic acid dimethylamide (142 mg, 36%). MS(ESI) *m/z* 435.3 $(M+H)^+$

Example 4

The Cell Titer-Glo Luminscent Cell Viability Assay (Promega# G7572) generates a luminescent signal that is proportional to the number of metabolically active cells present in a reaction, based on the quantitation of ATP. Cell

Titer-Glo Reagent was prepared by thawing a vial of Cell Titer-Glo Buffer in a 37°C water bath. The entire bottle of buffer was then added to the bottle of lyophilized Cell Titer-Glo Substrate provided in the kit. Lyophilized substrate was allowed to dissolve; the solution was then mixed by inversion and was ready for use. Jeko-1 cells were diluted to a density of 200,000 cells/mL and cultured in T250 flask. Before treatment (time 0), 3 x 100 micro liter aliquots were removed and placed into a black 96 well plate with clear bottom (Costar#3904). 50 uL of CTG reagent was added to each well. The plate was placed on an Orbital Shaker, protected from light and incubated using setting 4 for 30 minutes at RT. The plate was then read using the Envision Luminometer, and results exported. The cells remaining in the T250 flasks were either left untreated or treated with single agents or in combinations. The concentration of CDK4/6 inhibitor used was 100 nM and those of mTOR inhibitor used were 1, 2.5 and 5 nM. Plates were allowed to incubate for 72 hrs at 37°C and 5% $CO_2$. After 72hrs, 3 x 100 uL aliquots were removed and subjected to CTG as described above. Results were exported and analyzed using Microsoft Excel. The percentage of viable cells as compared to control growth was calculated using following equation:

$$\text{If } A>B\text{, then } 100 \times ((A-B)/(C-B))\text{, if not then } 100 \times (A-B/B)$$

Where:

A is the CTG read under treatment condition
B is the CTG read for Time 0 cells
C is CTG read for 72hr untreated cells

Example 5

To evaluate whether the CDK4/6 and mTOR inhibitor combination leads to more pronounced growth inhibition compared to the growth inhibitions observed with single agents, Jeko-1 mantle cell lymphoma cells were treated with 100 nM of CDK4/6 inhibitor, 1, 2.5 and 5 nM of mTOR inhibitor and the combinations of the two inhibitors, as shown in Figure 1. Growth inhibitions are measured using the CellTiter-Glo kit of Example 4. % growth of the treated cells, compared to the vehicle control, was obtained. As shown in figure 1, the treatment with 100 nM of CDK4/6 inhibitor led to 70% cell growth compared to the vehicle control, while the treatments with mTOR inhibitor alone led to approximately 30% growth at all three concentrations tested. Notably, the combinations of CDK4/6 and mTOR inhibitor led to much more pronounced growth inhibitions at all combinations tested. For example, less than 10% cell growth was observed for the 100 nM/5 nM CDK4/6 and mTOR inhibitor combination. This shows that CDK4/6 and mTOR inhibitor combination induces higher amounts of cell growth inhibitions, when evaluated against the single agent activities of the individual compound.

Example 6

To determine if CDK4/6 and mTOR inhibitor combinations resulted in synergistic growth inhibitions, we generated isobolograms, where we compared the actual growth inhibition values in combinations, to 25, 50 and 75% growth inhibitions predicted for additivity (Tallarida RJ (2006) An overview of drug combination analysis with isobolograms. Journal of Pharmacology and Experimental Therapeutics; 319 (1):1-7). Briefly, 9 titrating concentration points including 0 nM that yielded growth inhibition values that ranged from 0 to 100% as single agents were determined for both CDK4/6 and mTOR inhibitor. In a 96 well plate, the 9 concentration points for each agent were mixed in a matrix format, generating 81 combinations. This plate was used to treat Jeko-1 cells, and the resulting growth inhibition values were used to generate $IC_{50}$ values for the single agents and combinations. Graph was generated with CDK4/6 inhibitor concentrations shown on the y-axis and mTOR inhibitor concentrations shown on the x-axis. A straight line connecting the CDK4/6 inhibitor and the mTOR inhibitor $IC_{50}$ values represented growth inhibitions that were strictly additive for the combinations. Plots placed below the line of additivity (more growth inhibition) represented synergistic growth inhibitions, while plots above the line of additivity (less growth inhibition) represented antagonistic growth inhibitions.

Example 7

To evaluate whether the cell growth inhibition by the CDK4/6 and mTOR inhibitor combination is synergistic, we measured the single agent and combination activities in Jeko-1 cells and analyzed them using the isolobologram analysis prepared according to Example 6. Briefly, the single agent activities of CDK4/6 and mTOR inhibitors were measured to determine 9 titrating concentration points that would give 0 to 100% growth inhibitions for each agent. In a matrix format, all possible combinations for the 9 concentration points of each inhibitor were co-administered to Jeko-1 cells and the observed growth inhibitions were recorded. The concentrations that gave 50% growth inhibitions were then calculated for each compound and the combinations, and used to generate the graph shown in figure 2. Axis X and Y represent mTOR and CDK4/6 inhibitor concentrations, respectively. Line 1 represents the growth inhibitions predicted for additivity, when considering 50% growth inhibitions. Line 2 is the plot generated for the observed combinations concentrations that gave the 50% growth inhibitions, and it is profoundly placed below the line of additivity, suggesting a strong synergy for the growth inhibition. In summary, the CDK4/6 and mTOR inhibitor combination inhibited cell growth in synergistic manner in Jeko-1 mantle cell lymphoma cells.

Example 8

The synergistic effect in a breast cancer cell line MDA-MB453 by the CDK4/6 and mTOR inhibitor combination was also analyzed using an isoloblogram analysis as described in Example 7 above. Also in accordance with Example 7, the CDK4/6 and mTOR inhibitor combination inhibited cell growth in synergistic manner in MDA-MB453 breast cancer cells.

Example 9

A Jeko-1 xenograft model was used to measure anti-tumor activity in a 35 day treatment period of Compound A1, Compound B1, and the combination of Compounds A1 and B1. Significant anti-tumor activity was observed. When dosing was stopped and tumors were allowed to re-grow, the combination of Compounds A1 and B1 significantly delayed tumor growth by 20 days. In this model, both Compound A1 and Compound B1 had anti-tumor activity. However, the combination of Compounds A1 and B1 significantly extended tumor growth delay when treatment was stopped. See Figure 4.

Example 10

The PANC-1 pancreatic carcinomas used for implantation were maintained by serial engraftment in nude mice. To initiate tumor growth, a 1 mm3 fragment was implanted subcutaneously in the right flank of each test animal. Tumors were monitored twice weekly and then daily as their mean volume approached 100-150 mm3. Twenty two days after tumor cell implantation, on D1 of the study, the animals were sorted into four groups of ten mice, with individual tumor sizes of 108-221 mm3 and group mean tumor sizes of 150-153 mm3. Tumor size, in mm3, was calculated from:

$$\text{Tumor Volume} = (w2 \times l)/2$$

where w = width and *l* = length, in mm, of the tumor. Tumor weight can be estimated with the assumption that 1 mg is equivalent to 1 mm3 of tumor volume.

Group 1 mice received the Compound A1 and Compound B1 vehicles, and served as controls for all analyses. Groups 2 and 3 received monotherapies with 250 mg/kg qd, po x 21 days of Compound A1 or 10 mg/kg qd, po x 21 days of Compound B1. Group 4 received the combination therapy of Compound A1 and Compound B1.

Each animal was euthanized when tumor volume reached 1200mm3, or on the last day of the study (D55). For each animal whose tumor reached the endpoint volume, the time to endpoint (TTE) was calculated by the following equation:

$$\text{TTE} = (\log_{10} (\text{endpoint volume})\text{-b})/\text{m}$$

Where TTE is expressed in days, endpoint volume is in mm3, b is the intercept, and m is the slope of the line obtained by linear regression of a log-transformed tumor growth data set. The data set is comprised of the first observation that exceeded the study endpoint volume and the three consecutive observations that immediately preceded the attainment of the endpoint volume. The calculated TTE is usually less than the day on which an animal is euthanized for tumor size. An animal with a tumor that did not reach the endpoint is assigned a TTE value equal to the last day. An animal classified as having died from TR causes or non-treatment-related metastasis (NTRm) is assigned a TTE value equal to the day of death. An animal classified as having died from NTR causes is excluded from TTE calculations.

Treatment efficacy was determined from tumor growth delay (TGD), which is defined as the increase in the median TTE for a treatment group compared with the control group: TGD = T - C, expressed in days, or as a percentage of the median TTE of the control group: %TGD = [(T - C)/C] x 100, where: T = median TTE for a treatment group, C = median TTE for the designated control group.

These studies demonstrate that neither Compound A1 nor Compound B1 had significant anti-tumor activity in the PANC-1 xenograft model. However, the combination of Compounds A1 and B1 resulted in tumor stasis (Figure 5A) and significantly delayed tumor regrowth by 18 days (Figure 5B).

Example 11

Potential synergistic interactions between CDK4/6 and mTOR inhibitor combinations were assessed relative to the Loewe additivity model using CHALICE software, via a synergy score calculated from the differences between the observed and Loewe model values across the response matrix. Briefly, 9 titrating concentration ranging from 10uM diluted serially three folds for CDK4/6 inhibitors and 0.1uM diluted serially 3 folds for the mTOR inhibitors, including 0uM, were used. In a 96 well plate, the 9 concentration points for each agent were mixed in a matrix format, generating 81 combinations. This plate was used to treat Jeko-1 cells, and the resulting inhibition values were used by CHALICE software to generate Inhibition and ADD Excess Inhibition matrices, as well as the isobolograms. A more detailed explanation of the technique and calculation can be found in Lehar et al. "Synergistic drug combinations improve therapeutic selectivity", Nat. Biotechnol. 2009, July; 27(7), 659-666Inhibition matrix shows the actual inhibition observed by the CTG assay at the respective concentrations of the compounds. ADD Excess inhibition shows the excess inhibition observed over the inhibition predicted by the Loewe additivity model. In addition to the matrices, one can use isobolograms to observe synergy. The inhibition level for each isobologram was chosen manually so as to observe the best synergistic effects. Isobologram was generated with CDK4/6 inhibitor concentrations shown on the y-axis and mTOR inhibitor concentrations shown on the x-axis. A straight line connecting the CDK4/6 inhibitor and the mTOR inhibitor concentrations which produce the chosen level of inhibition represented growth inhibitions that were strictly additive for the combinations. Plots placed below the line of additivity (more growth inhibition) represented synergistic growth inhibitions, while plots above the line of additivity (less growth inhibition) represented antagonistic growth inhibitions.

Synergic interaction between the following pairs of CDK4/6 inhibitor and the mTOR inhibitor combination were studied, the synergy scores, and the corresponding figure illustrations are listed below. The combination of Compound A1 with Compound B1 falls within the definition of the present invention. Other combinations described below are reference examples.

| CDK4/6 inhibitor | mTOR inhibitor | Synergy Score | Figure |
|---|---|---|---|
| Compound A1 | Compound B1 | 4.92 | 6 |
| Compound A1 | Compound B2 | 7.77 | 7 |
| Compound A4 | Compound B1 | 7.16 | 8 |
| Compound A2 | Compound B1 | 3.76 | 9 |
| Compound A3 | Compound B1 | 7.1 | 10 |
| Compound A6 | Compound B1 | 6.41 | 11 |
| Compound A5 | Compound B1 | 4.04 | 12 |
| Compound A4 | Compound B2 | 5.73 | 13 |
| Compound A2 | Compound B2 | 4.57 | 14 |
| Compound A3 | Compound B2 | 6.85 | 15 |
| Compound A6 | Compound B2 | 3.24 | 16 |
| Compound A5 | Compound B2 | 5.86 | 17 |

## Claims

1. A combination comprising a first agent that is a cyclin dependent kinase 4 or cyclin dependent kinase 6 (CDK4/6) inhibitor, and a second agent that is an mTOR inhibitor, wherein the first agent is 7-Cyclopentyl-2-(5-piperazin-1-yl-pyridin-2-ylamino)-7H-pyrrolo[2,3-d]pyrimidine-6-carboxylic acid dimethylamide, or a pharmaceutically acceptable salt thereof, and the second agent is everolimus.

2. The combination according to claim 1 for use in the treatment of cancer.

3. The combination for use according to claim 2, wherein the cancer is dependent on the CDK4, CDK6 or mTOR pathway.

4. The combination for use according to claim 2, wherein the cancer is a solid tumor cancer.

5. The combination for use according to claim 2, wherein the cancer is pancreatic cancer, breast cancer, mantle cell lymphoma, non small cell lung cancer, melanoma, colon cancer, esophageal cancer, liposarcoma, multiple myeloma, T-cell leukemia, renal cell carcinoma, gastric cancer, renal cell carcinoma, glioblastoma, hepatocellular carcinoma, gastric cancer, lung cancer or colon cancer.

6. The combination for use according to claim 2, wherein the cancer is pancreatic cancer, breast cancer, or mantle cell lymphoma.

7. The combination for use according to claim 2, wherein the cancer is a lymphoma.

8. The combination for use according to claim 2, wherein the first agent and the second agent are present in a combined dosage form.

9. The combination for use according to claim 2 wherein the first agent and the second agent are present in a separate dosage form.

**Patentansprüche**

1. Kombination, umfassend ein erstes Mittel, bei dem es sich um einen Inhibitor von cyclinabhängiger Kinase 4 oder cyclinabhängiger Kinase 6 (CDK4/6) handelt, und ein zweites Mittel, bei dem es sich um einen mTOR-Inhibitor handelt, wobei es sich bei dem ersten Mittel um 7-Cyclopentyl-2-(5-piperazin-1-ylpydin-2-ylamino)-7H-pyrrolo[2,3-d]pyrimidin-6-carbonsäuredimethylamid oder ein pharmazeutisch unbedenkliches Salz davon handelt und es sich bei dem zweiten Mittel um Everolimus handelt.

2. Kombination nach Anspruch 1 zur Verwendung bei der Behandlung von Krebs.

3. Kombination zur Verwendung nach Anspruch 2, wobei die Krebserkrankung vom CDK4-, CDK6- oder mTOR-Pfad abhängig ist.

4. Kombination zur Verwendung nach Anspruch 2, wobei es sich bei der Krebserkrankung um eine solide Tumorkrebserkrankung handelt.

5. Kombination zur Verwendung nach Anspruch 2, wobei es sich bei der Krebserkrankung um Bauchspeicheldrüsenkrebs, Brustkrebs, Mantelzelllymphom, nichtkleinzelligen Lungenkrebs, Melanom, Dickdarmkrebs, Speiseröhrenkrebs, Liposarkom, multiples Myelom, T-Zell-Leukämie, Nierenzellkarzinom, Magenkrebs, Nierenzellkarzinom, Glioblastom, hepatozelluläres Karzinom, Magenkrebs, Lungenkrebs oder Dickdarmkrebs handelt.

6. Kombination zur Verwendung nach Anspruch 2, wobei es sich bei der Krebserkrankung um Bauchspeicheldrüsenkrebs, Brustkrebs oder Mantelzelllymphom handelt.

7. Kombination zur Verwendung nach Anspruch 2, wobei es sich bei der Krebserkrankung um ein Lymphom handelt.

8. Kombination zur Verwendung nach Anspruch 2, wobei das erste Mittel und das zweite Mittel in einer kombinierten Dosierungsform vorliegen.

9. Kombination zur Verwendung nach Anspruch 2, wobei das erste Mittel und das zweite Mittel in einer getrennten Dosierungsform vorliegen.

**Revendications**

1. Combinaison comprenant un premier agent qui est un inhibiteur de kinase cycline-dépendante 4 ou de kinase

cycline-dépendante 6 (CDK4/6), et un deuxième agent qui est un inhibiteur de mTOR, dans laquelle le premier agent est le diméthylamide d'acide 7-cyclopentyl-2-(5-pipérazin-1-yl-pyridin-2-ylamino)-7H-pyrrolo[2,3-d]pyrimidine-6-carboxylique, ou un sel pharmaceutiquement acceptable de celui-ci, et le deuxième agent est l'évérolimus.

**2.** Combinaison selon la revendication 1 pour utilisation dans le traitement d'un cancer.

**3.** Combinaison pour utilisation selon la revendication 2, le cancer étant dépendant de la voie CDK4, CDK6 ou mTOR.

**4.** Combinaison pour utilisation selon la revendication 2, le cancer étant un cancer à tumeur solide.

**5.** Combinaison pour utilisation selon la revendication 2, le cancer étant un cancer du pancréas, un cancer du sein, un lymphome à cellules du manteau, un cancer du poumon non à petites cellules, un mélanome, un cancer du côlon, un cancer de l'oesophage, un liposarcome, un myélome multiple, une leucémie à cellules T, un carcinome à cellules rénales, un cancer de l'estomac, un carcinome à cellules rénales, un glioblastome, un carcinome hépatocellulaire, un cancer de l'estomac, un cancer du poumon ou un cancer du côlon.

**6.** Combinaison pour utilisation selon la revendication 2, le cancer étant un cancer du pancréas, un cancer du sein, ou un lymphome à cellules du manteau.

**7.** Combinaison pour utilisation selon la revendication 2, le cancer étant un lymphome.

**8.** Combinaison pour utilisation selon la revendication 2, dans laquelle le premier agent et le deuxième agent sont présents dans une forme pharmaceutique combinée.

**9.** Combinaison pour utilisation selon la revendication 2 dans laquelle le premier agent et le deuxième agent sont présents dans une forme pharmaceutique séparée.

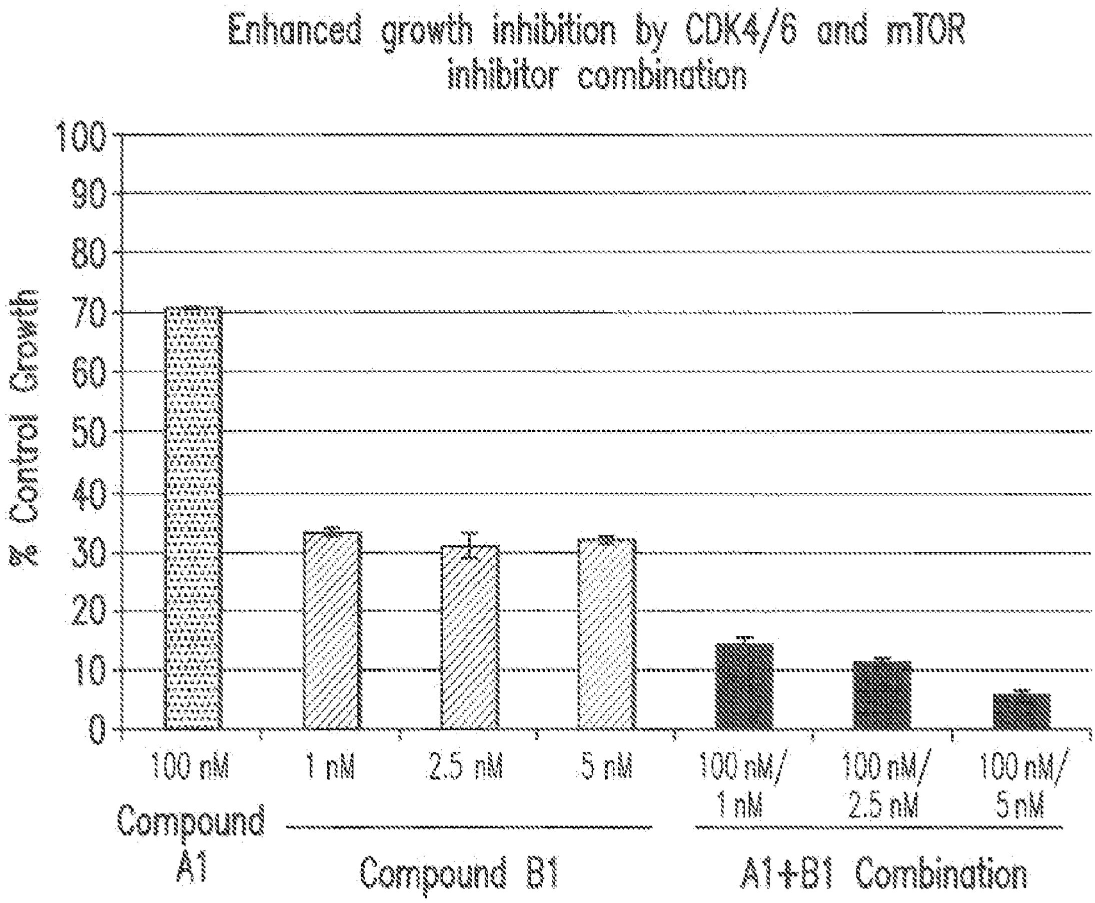
Enhanced growth inhibition by CDK4/6 and mTOR inhibitor combination
% Control Growth
100
90
80
70
60
50
40
30
20
10
0
100 nM
1 nM
2.5 nM
5 nM
100 nM/ 1 nM
100 nM/ 2.5 nM
100 nM/ 5 nM
Compound A1
Compound B1
A1+B1 Combination

FIG.1

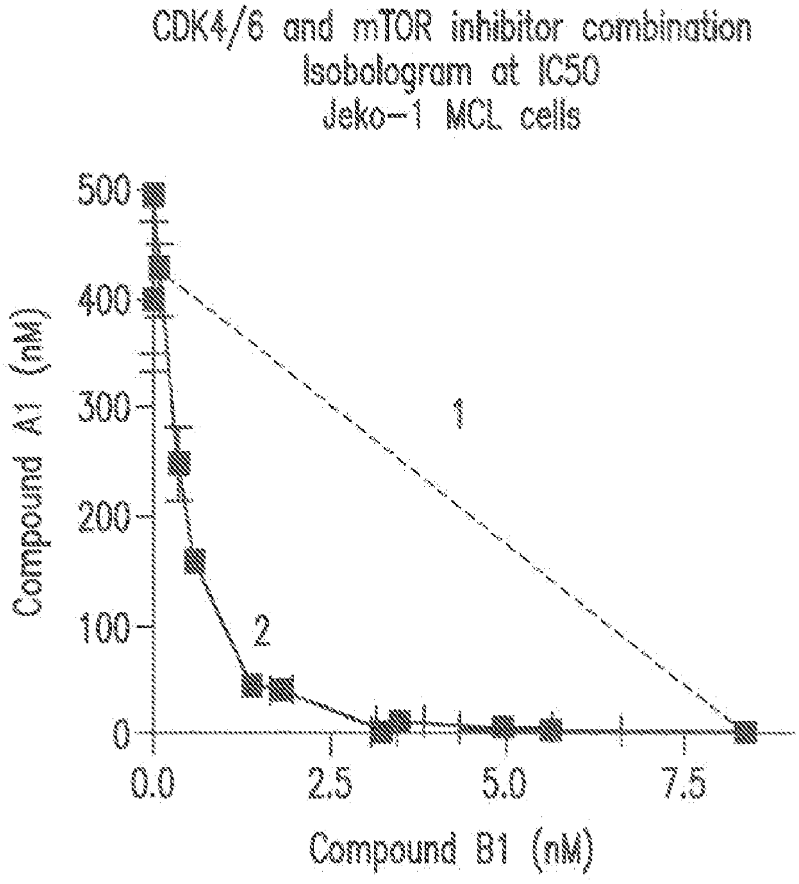
CDK4/6 and mTOR inhibitor combination
Isobologram at IC50
Jeko-1 MCL cells
500
400
300
200
100
0
Compound A1 (nM)
1
2
0.0
2.5
5.0
7.5
Compound B1 (nM)

FIG.2

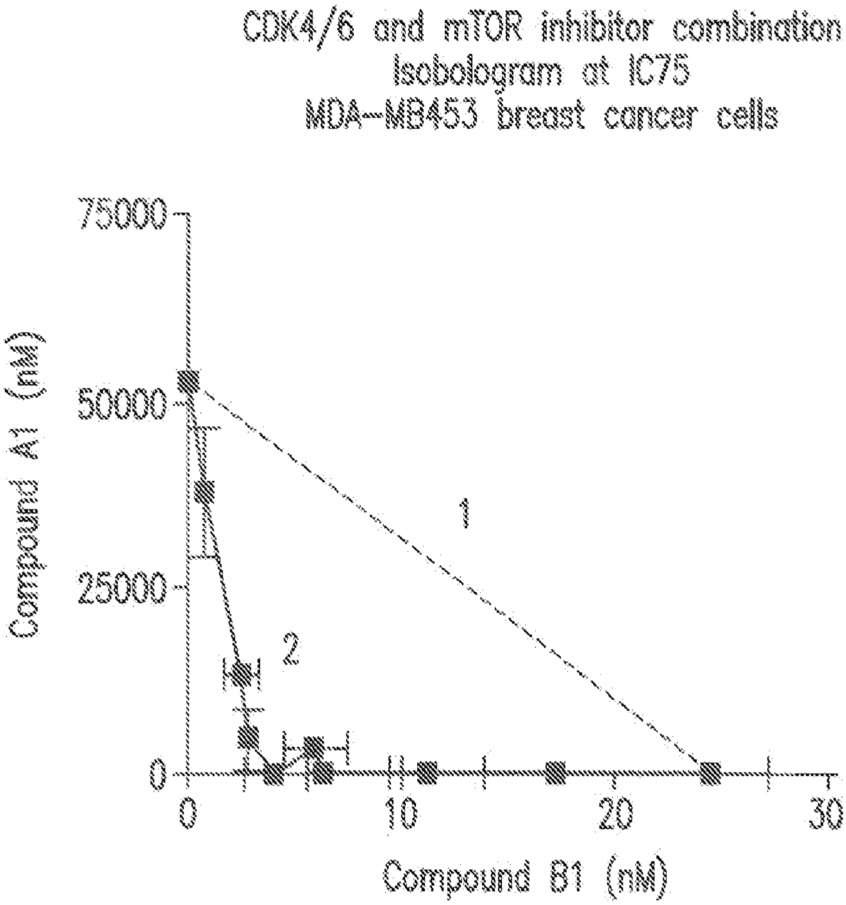
CDK4/6 and mTOR inhibitor combination
Isobologram at IC75
MDA-MB453 breast cancer cells
75000
50000
25000
0
Compound A1 (nM)
1
2
0
10
20
30
Compound B1 (nM)

FIG.3

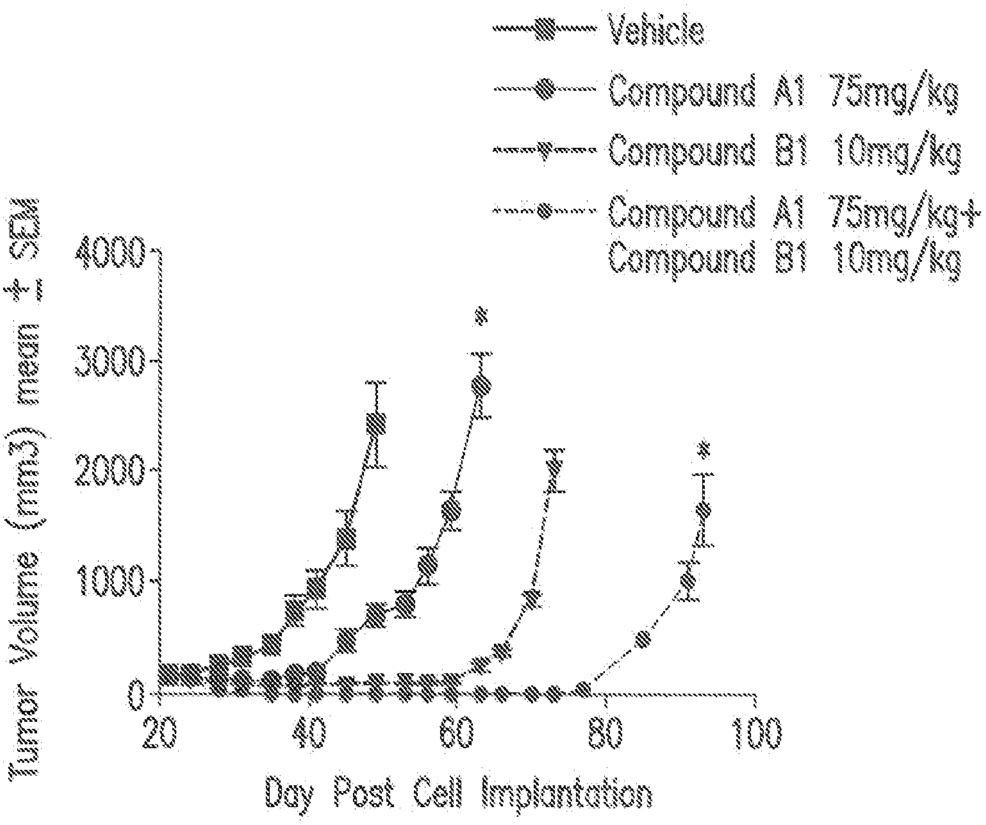
Vehicle
Compound A1 75mg/kg
Compound B1 10mg/kg
Compound A1 75mg/kg+
Compound B1 10mg/kg
Tumor Volume (mm3) mean ± SEM
4000
3000
2000
1000
0
20
40
60
80
100
Day Post Cell Implantation
*
*

FIG.4

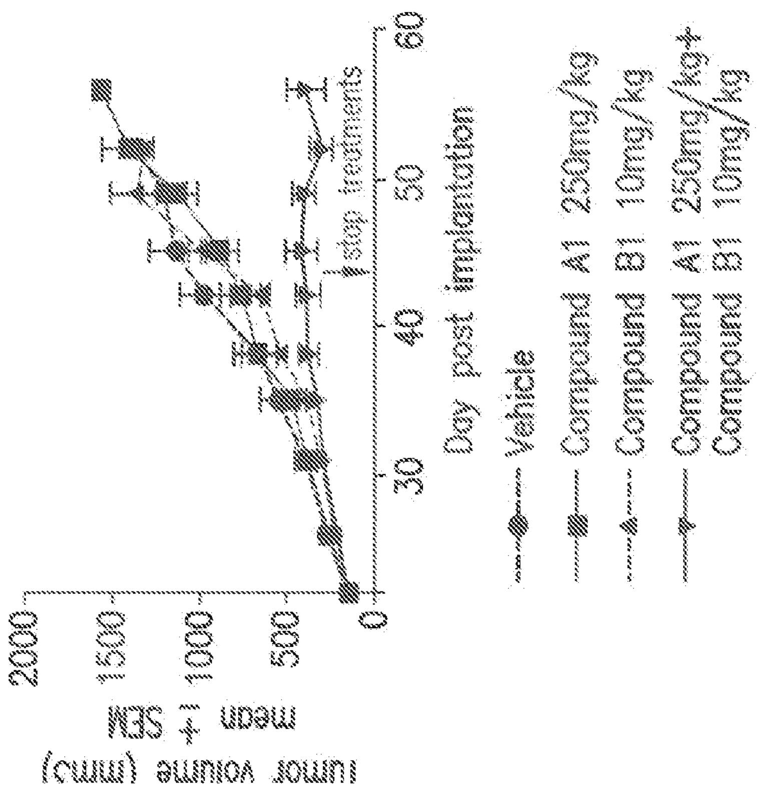
Tumor volume (mm3)
mean +/- SEM
2000
1500
1000
500
0
30
40
50
60
stop treatments
Day post implantation
Vehicle
Compound A1 250mg/kg
Compound B1 10mg/kg
Compound A1 250mg/kg+
Compound B1 10mg/kg

FIG.5A

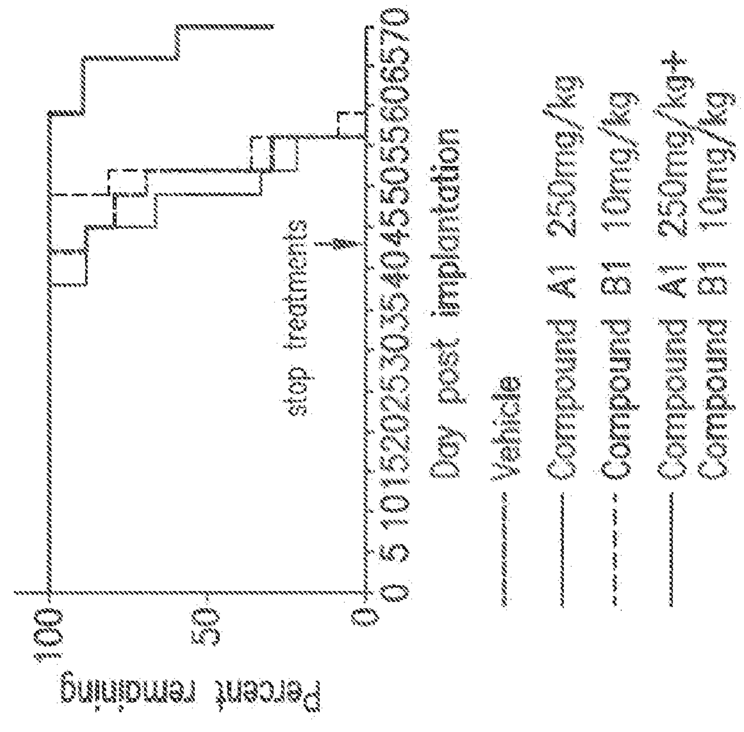
Percent remaining
100
50
0
0 5 101520253035404550556065 70
stop treatments
Day post implantation
Vehicle
Compound A1 250mg/kg
Compound B1 10mg/kg
Compound A1 250mg/kg+
Compound B1 10mg/kg

FIG.5B

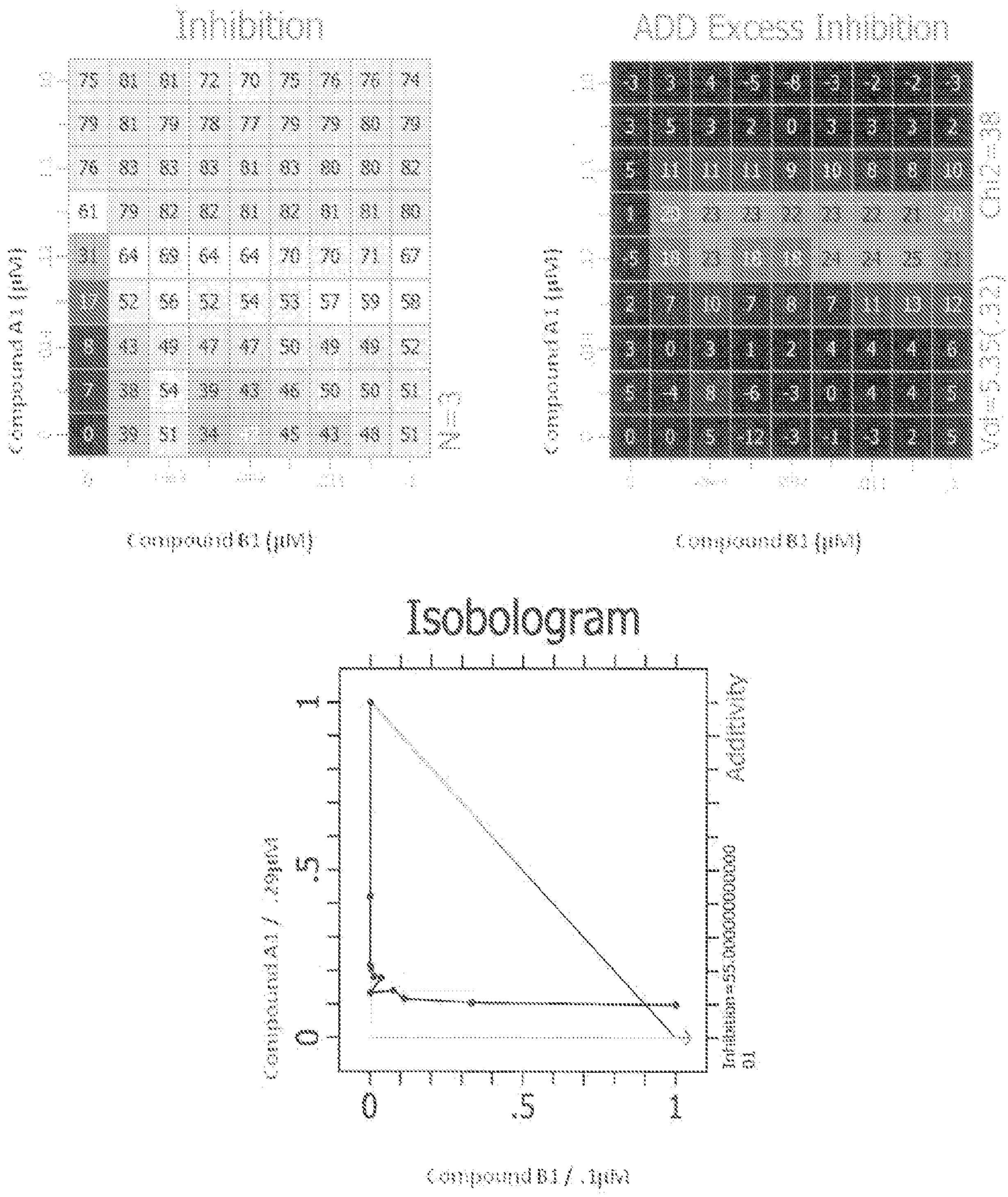
Inhibition
Compound A1 (µM)
Compound B1 (µM)
N=3
ADD Excess Inhibition
Chi2 =38
Vol=5.35(.32)
Compound A1 (µM)
Compound B1 (µM)
Isobologram
Compound A1 / .29µM
Compound B1 / .1µM
Additivity
Inhibition=55.000000000000
0
.5
1

FIG. 6

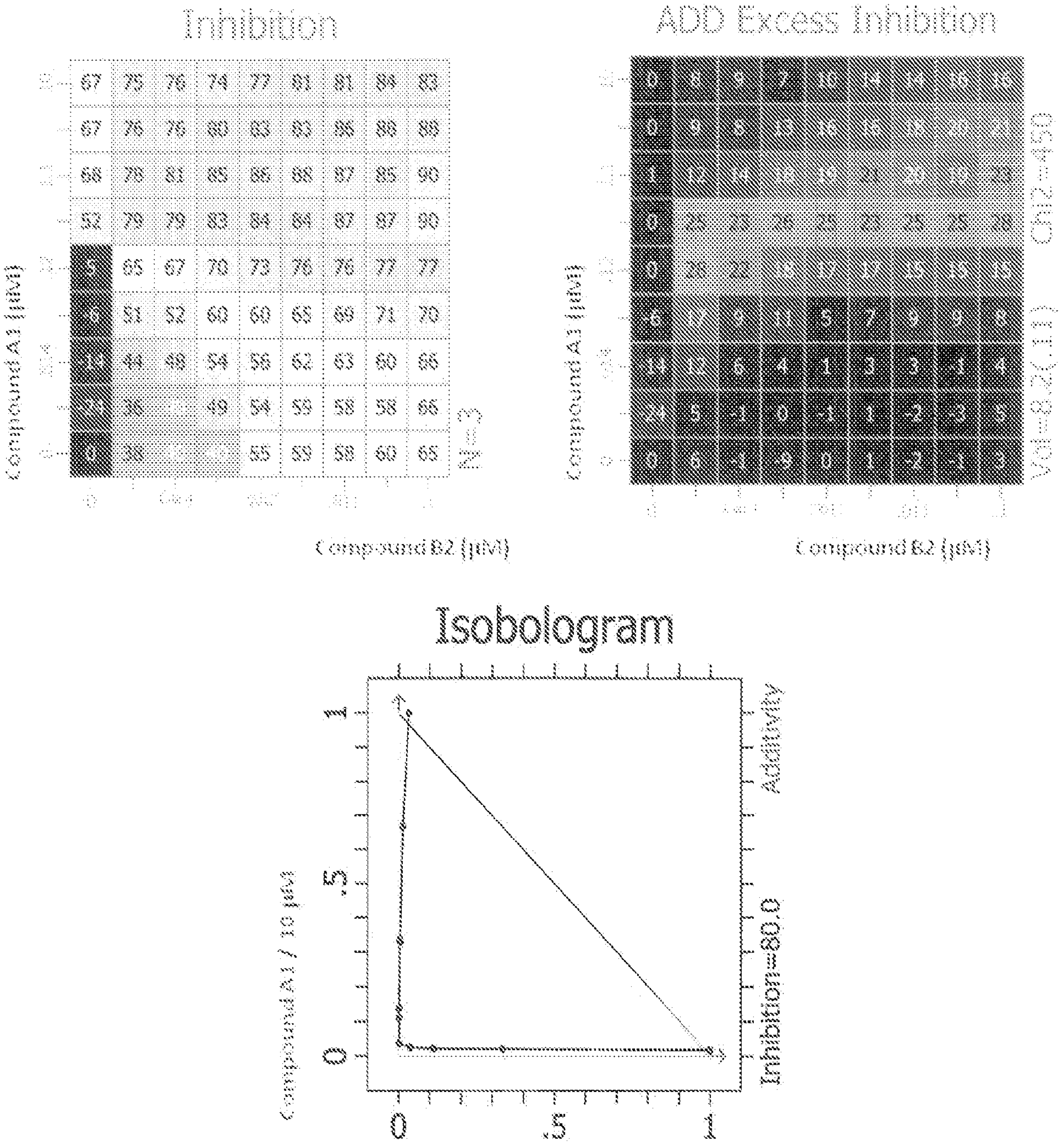
Inhibition
N=3
Compound A1 (μM)
Compound B2 (μM)
ADD Excess Inhibition
Chi2=450
Compound A1 (μM)
Compound B2 (μM)
Isobologram
Additivity
Inhibition=80.0
Compound A1 / 10 μM
Compound B2/.1 μM
0
.5
1

FIG. 7

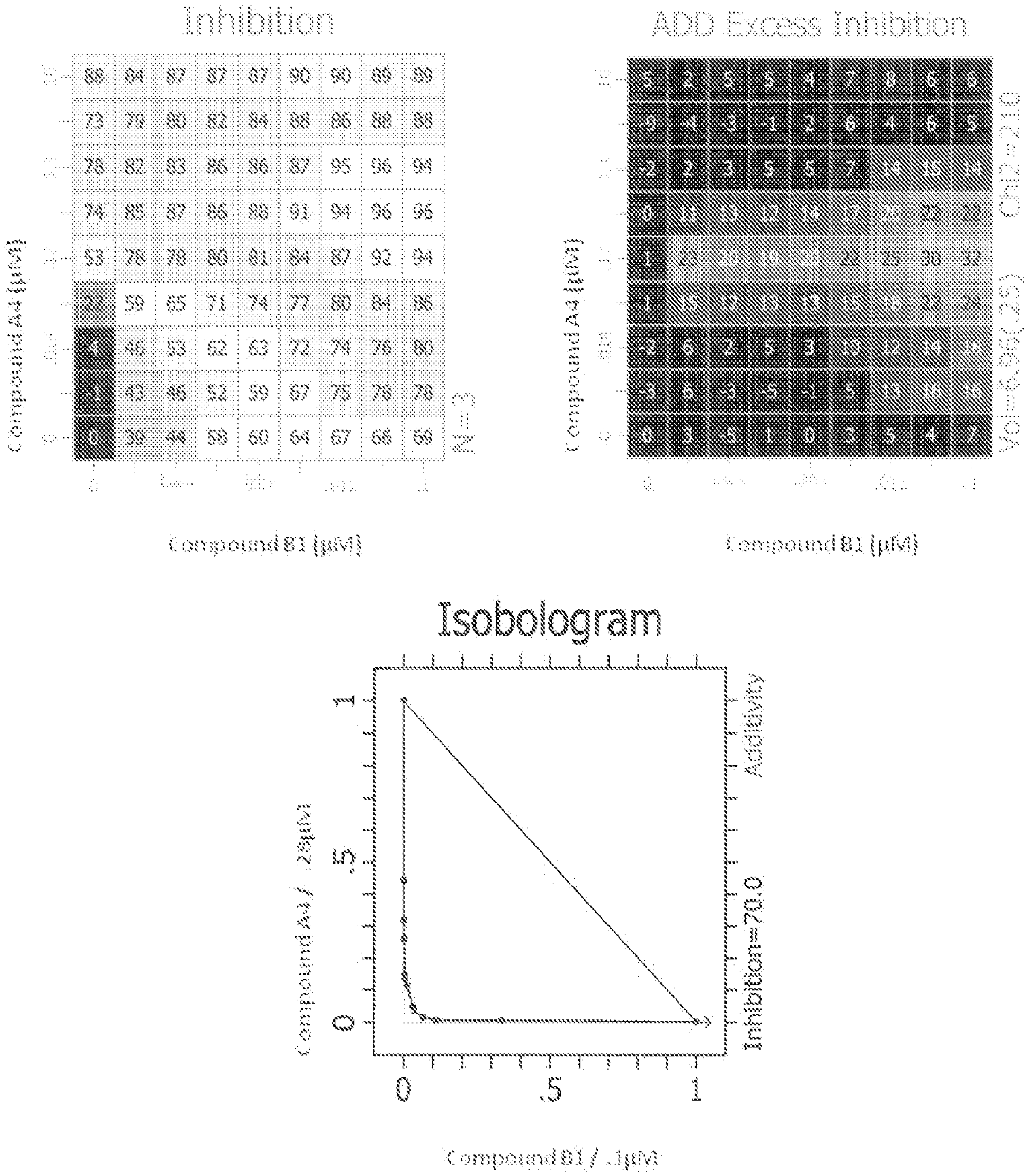
Inhibition
88 84 87 87 87 90 90 89 89
73 79 80 82 84 88 86 88 88
78 82 83 86 86 87 95 96 94
74 85 87 86 88 91 94 96 96
53 78 78 80 81 84 87 92 94
22 59 65 71 74 77 80 84 86
4 46 53 62 63 72 74 76 80
1 43 46 52 59 67 75 78 78
0 39 44 58 60 64 67 66 69
N=3
Compound A4 [µM]
Compound B1 [µM]
ADD Excess Inhibition
Chi2=210
Vol=6.96(.25)
Compound A4 [µM]
Compound B1 [µM]
Isobologram
Additivity
Inhibition=70.0
Compound A4 / .28µM
Compound B1 / .1µM
0
.5
1

FIG. 8

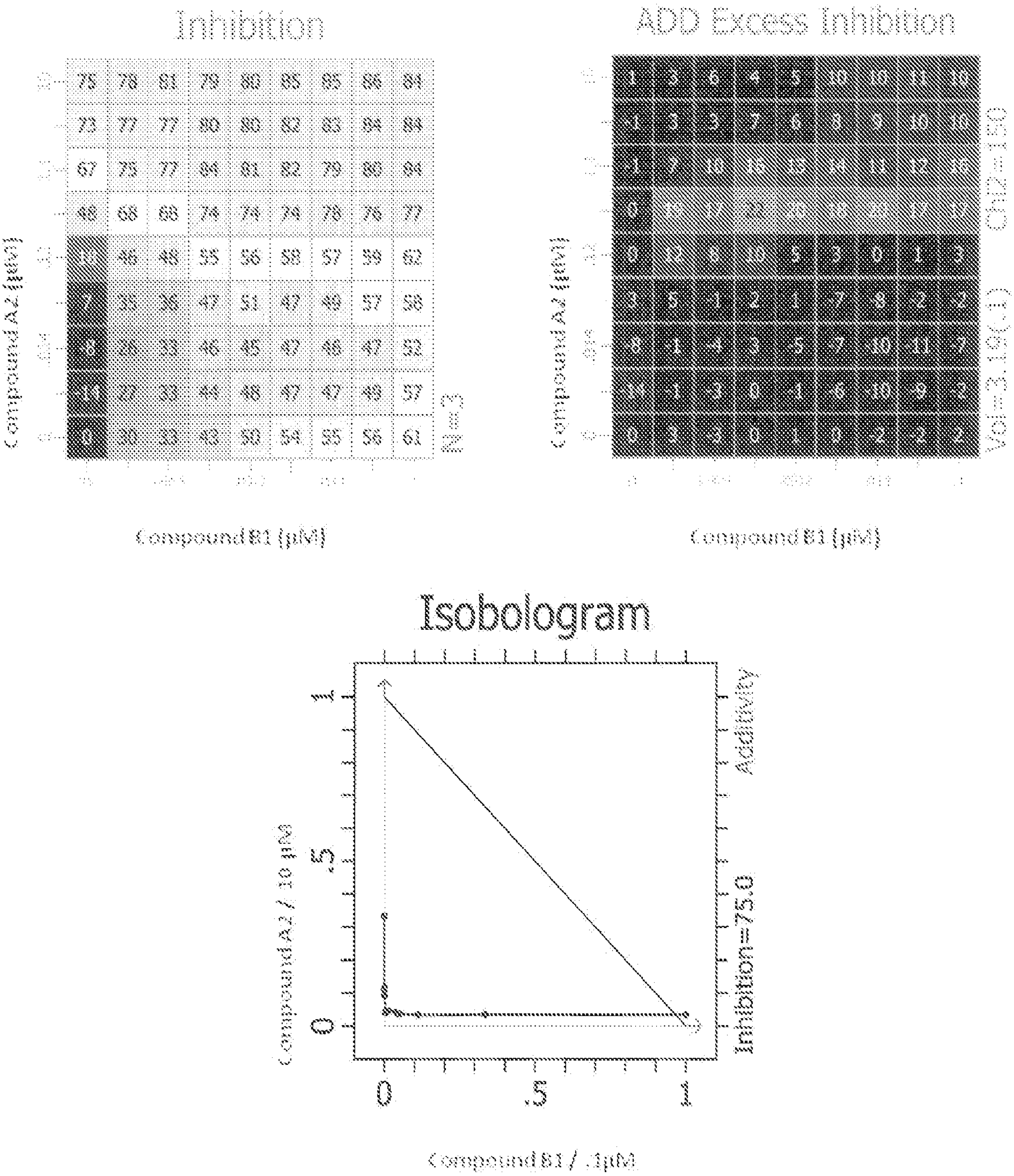
Inhibition
N=3
Compound A2 (µM)
Compound B1 (µM)
ADD Excess Inhibition
Chi2=150
Vol=3.19(.1)
Compound A2 (µM)
Compound B1 (µM)
Isobologram
Additivity
Inhibition=75.0
Compound A2 / 10 µM
Compound B1 / .1µM
0
.5
1

FIG. 9

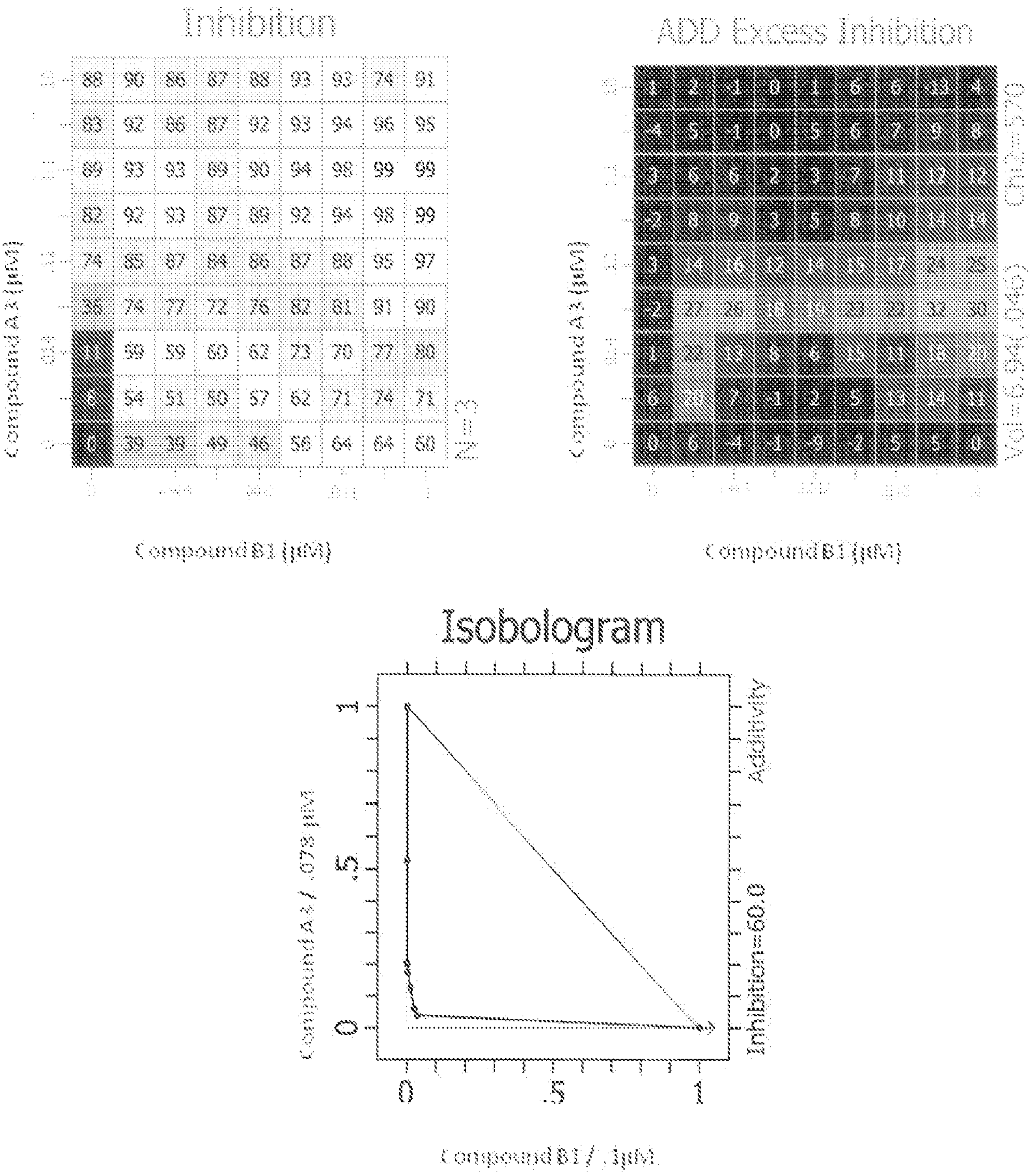
Inhibition
Compound A3 (µM)
Compound B1 (µM)
N=3
ADD Excess Inhibition
Compound A3 (µM)
Compound B1 (µM)
Chi2=570
Vol=6.94(.046)
Isobologram
Compound A3 / .073 µM
Compound B1 / .1µM
Additivity
Inhibition=60.0
0
.5
1

FIG. 10

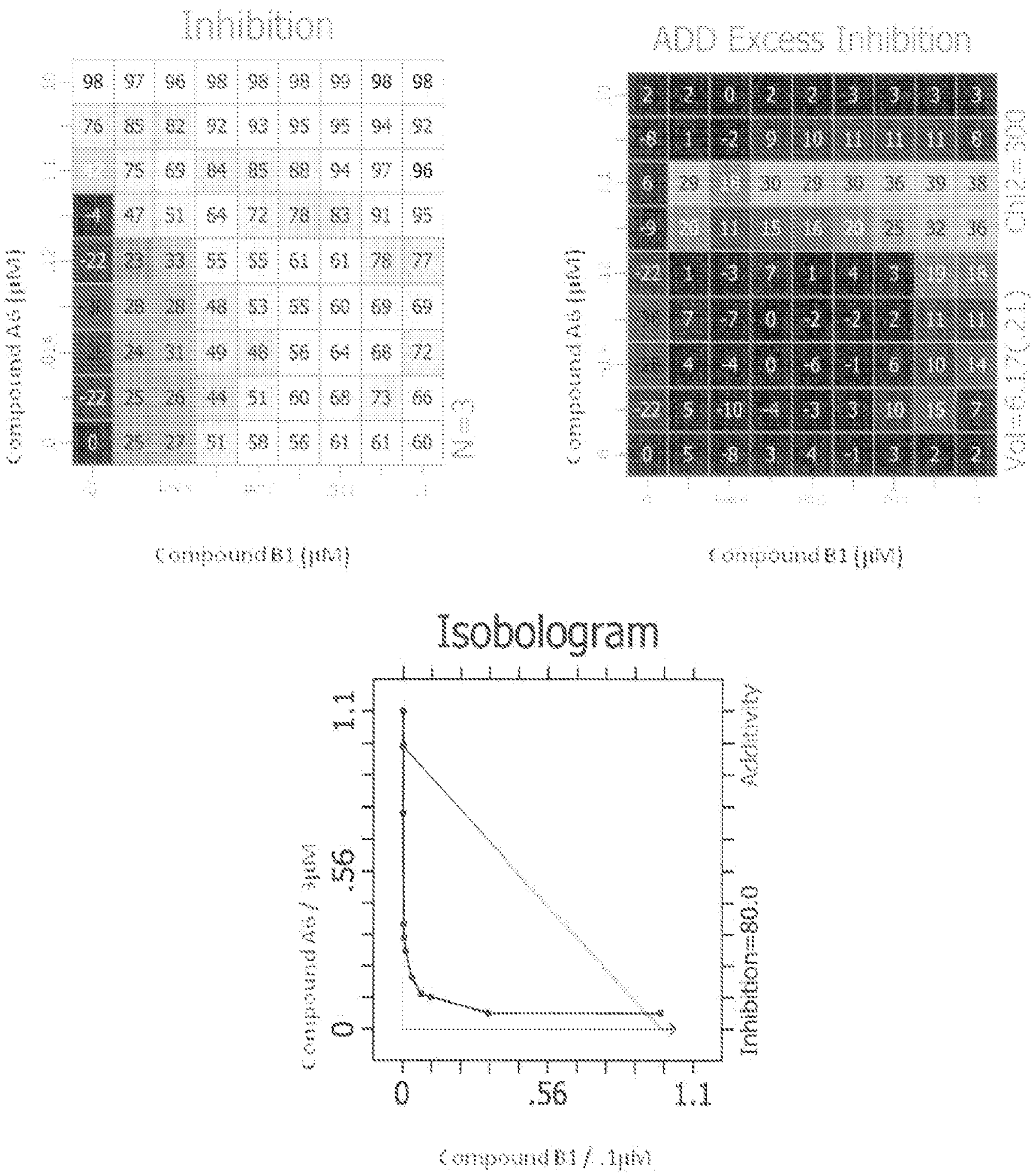
Inhibition
Compound A5 [μM]
Compound B1 [μM]
N=3
ADD Excess Inhibition
Compound A5 [μM]
Compound B1 [μM]
Chi2 = 300
Isobologram
Compound A5 / μM
Compound B1 / .1μM
Additivity
Inhibition=80.0
0
.56
1.1

FIG. 11

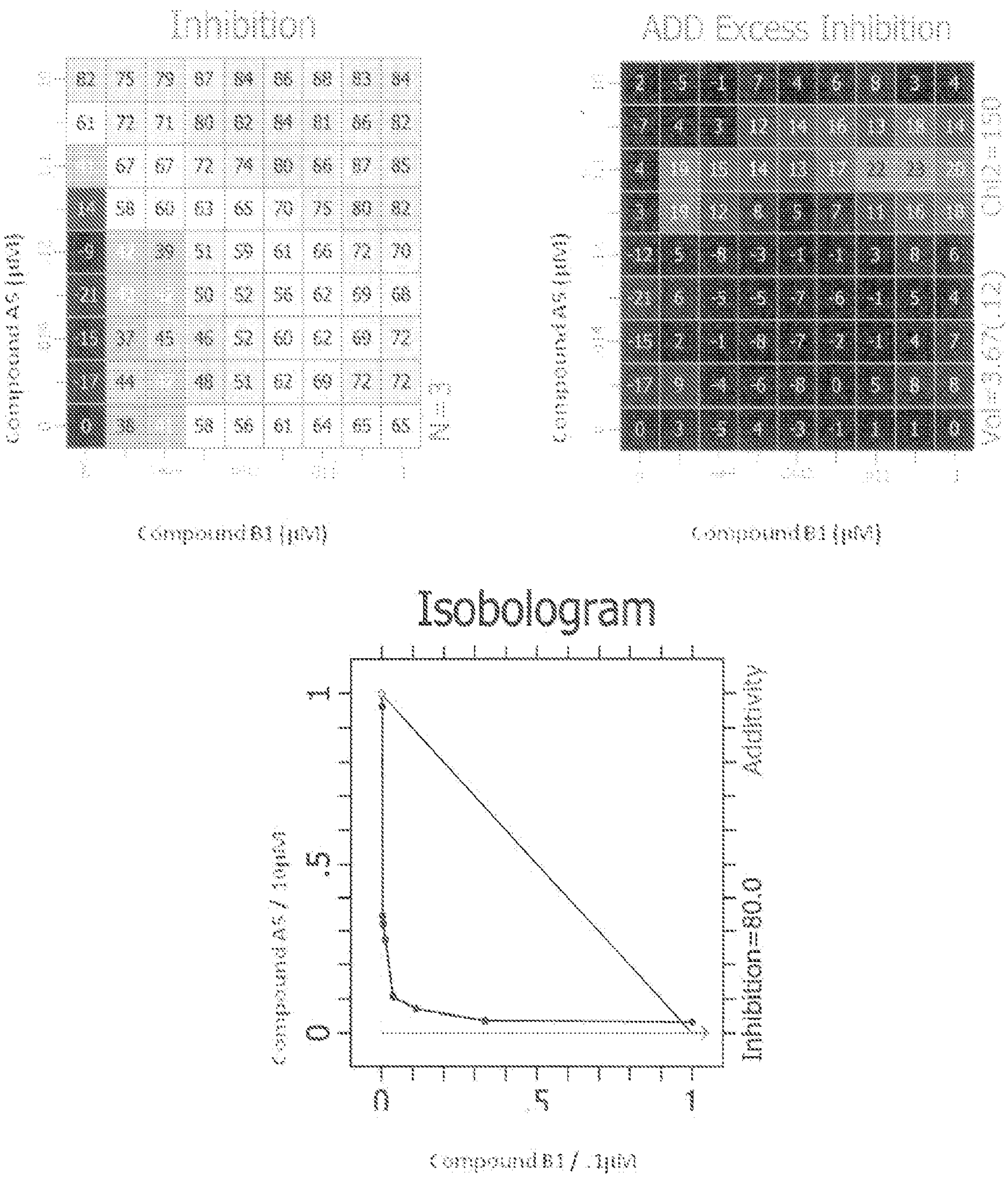
Inhibition
Compound A5 (µM)
Compound B1 (µM)
N=3
ADD Excess Inhibition
Compound A5 (µM)
Compound B1 (µM)
Chi2 = 150
Vol=3.67(.12)
Isobologram
Compound A5 / 10µM
Compound B1 / .1µM
Additivity
Inhibition=80.0
0
.5
1

FIG. 12

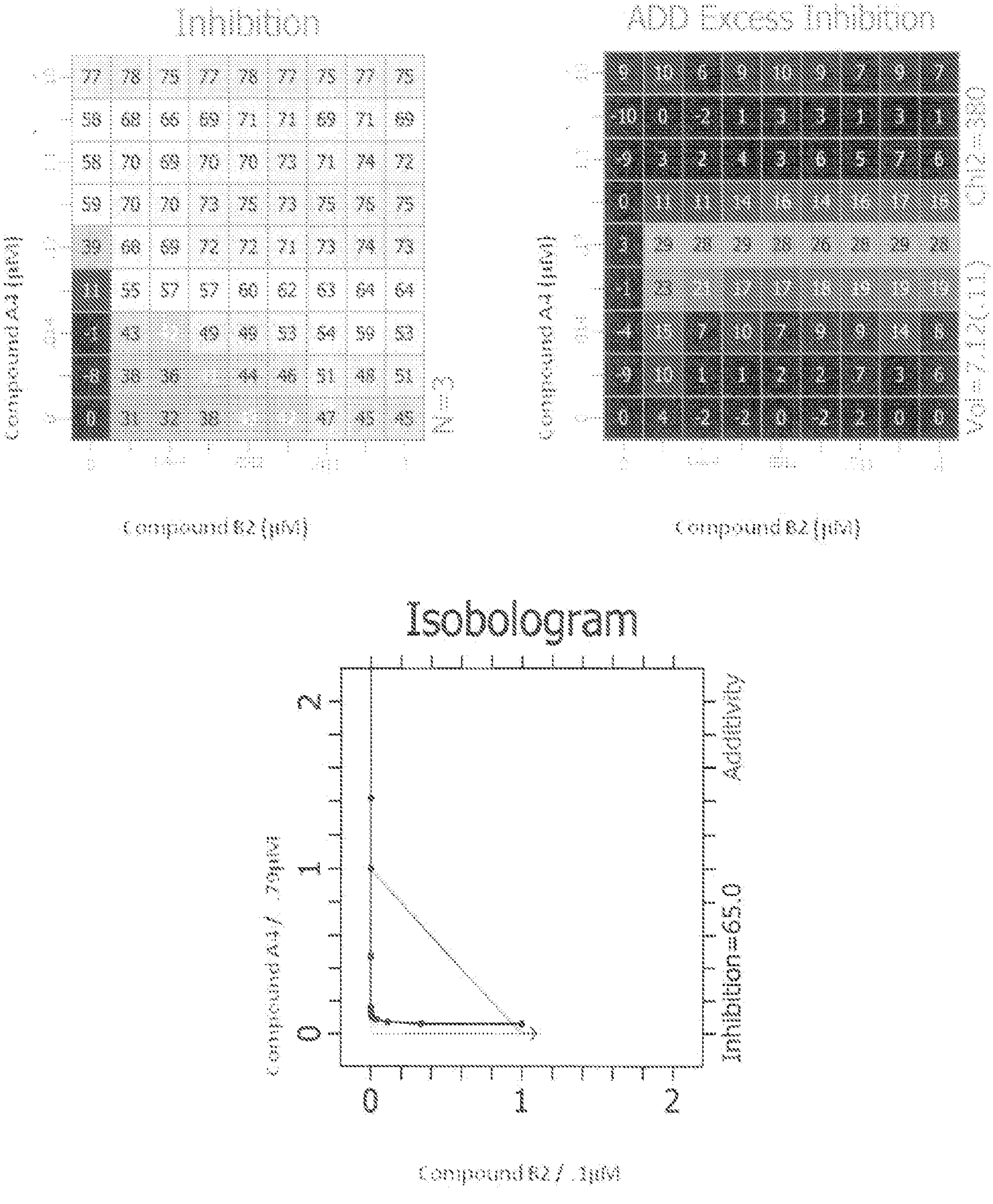
Inhibition
Compound A4 (µM)
Compound B2 (µM)
N=3
ADD Excess Inhibition
Chi2 = 380
Vol = 7.12(.11)
Compound A4 (µM)
Compound B2 (µM)
Isobologram
Additivity
Inhibition=65.0
0
1
2
0
1
2

FIG. 13

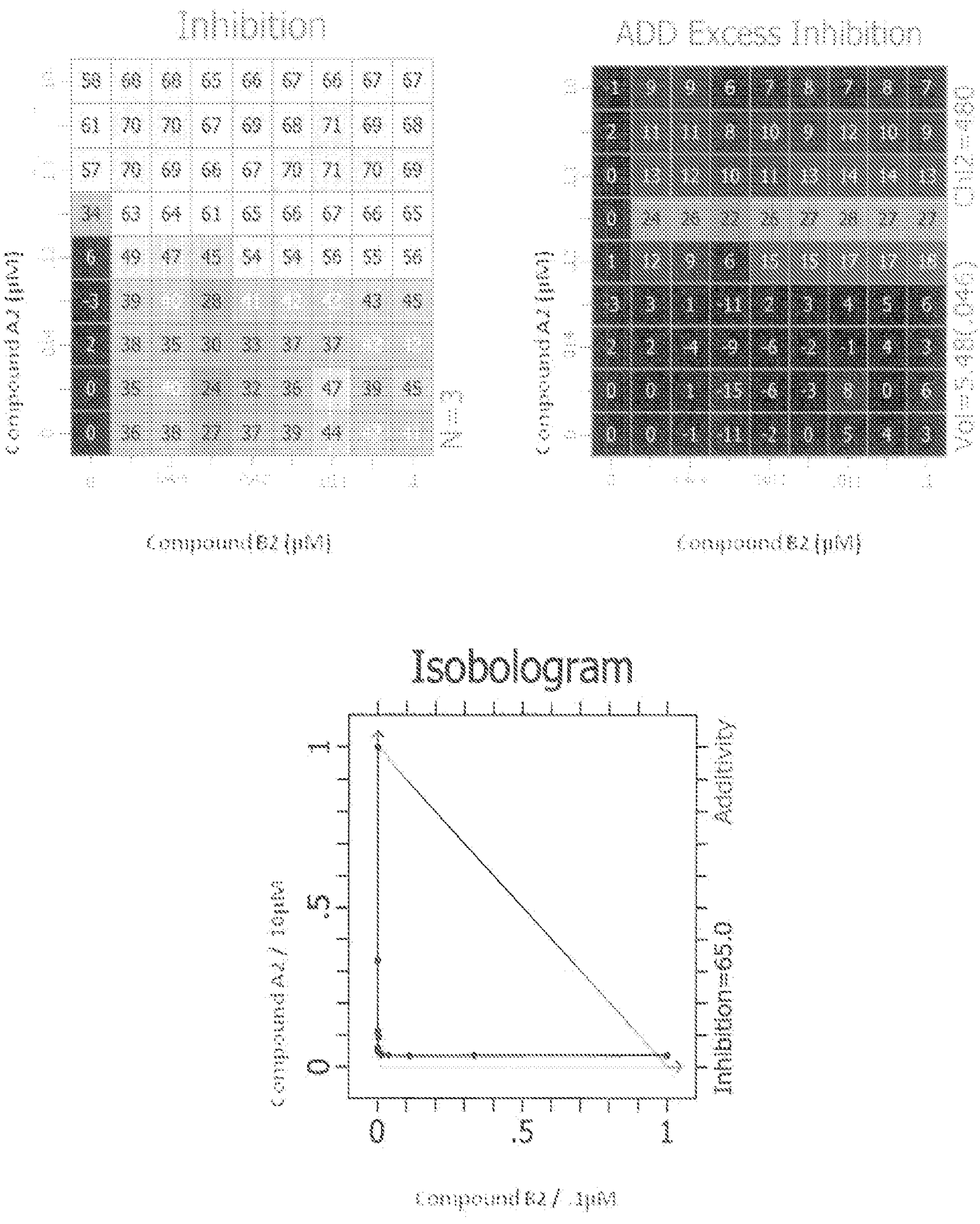
Inhibition
ADD Excess Inhibition
Compound A2 (µM)
Compound B2 (µM)
N=3
Chi2=480
Vol=5.48(.046)
Isobologram
Compound A2 / 10µM
Compound B2 / .1µM
Additivity
Inhibition=65.0
0
.5
1

FIG. 14

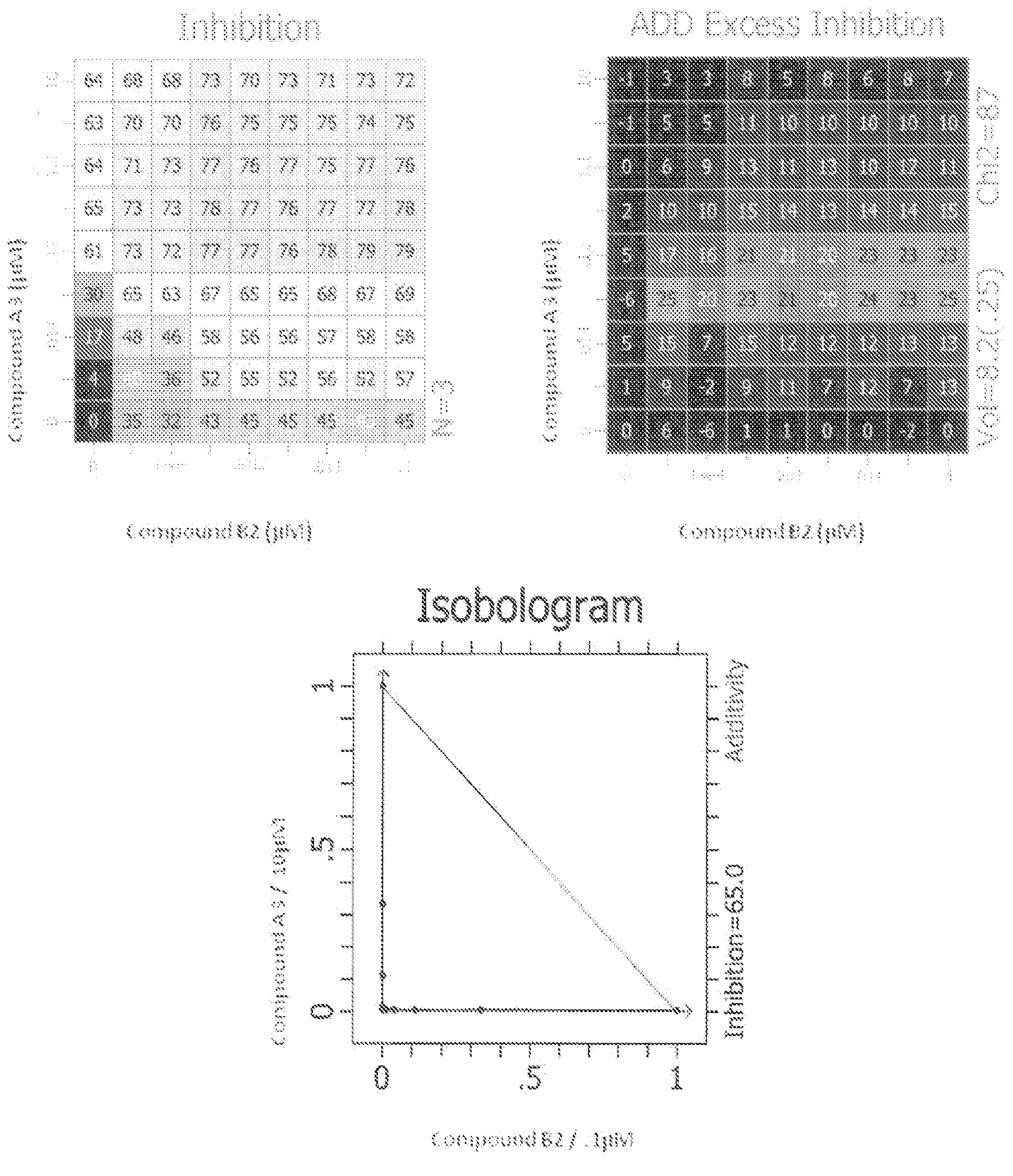
Inhibition
Compound A3 (µM)
Compound B2 (µM)
N=3
ADD Excess Inhibition
Compound A3 (µM)
Compound B2 (µM)
Chi2=87
Vol=8.2(.25)
Isobologram
Compound A3 / 10µM
Compound B2 / .1µM
Additivity
Inhibition=65.0

FIG. 15

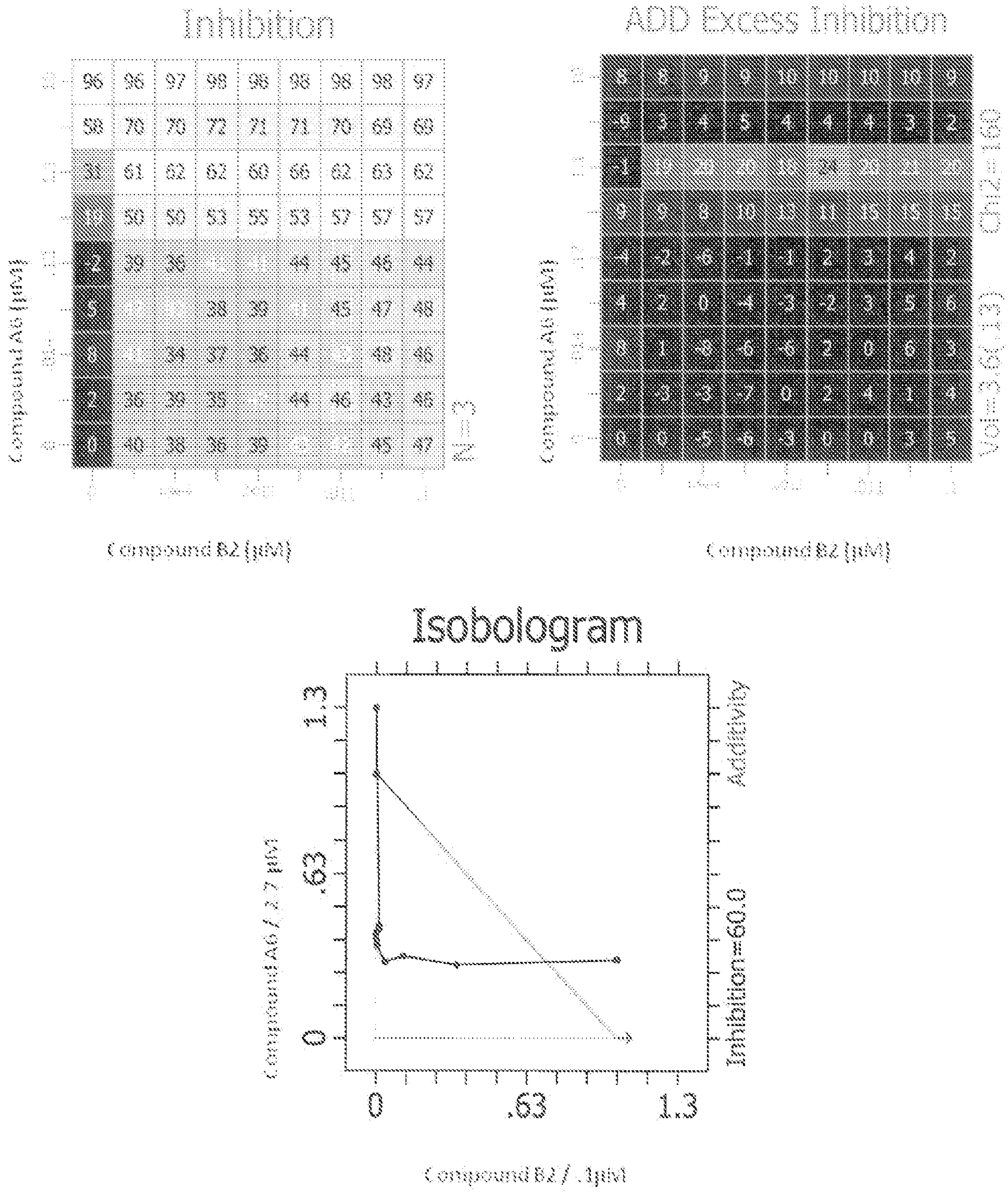
Inhibition
N=3
Compound A6 (μM)
Compound B2 (μM)
ADD Excess Inhibition
Chi2=160
Vol=3.6(.13)
Compound A6 (μM)
Compound B2 (μM)
Isobologram
1.3
.63
0
Compound A6 / 2.7 μM
Additivity
Inhibition=60.0
0
.63
1.3
Compound B2 / .1μM

FIG. 16

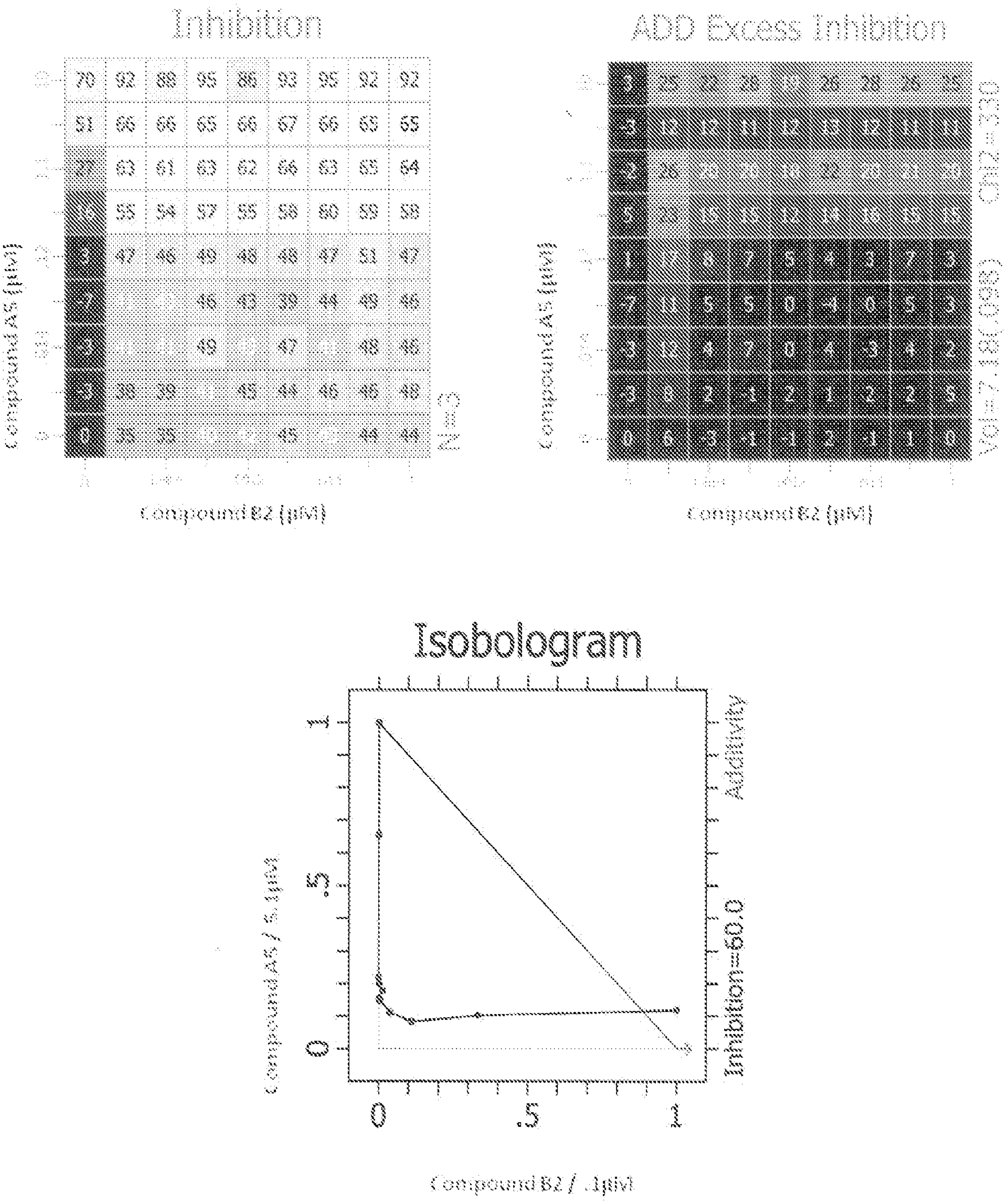
Inhibition
Compound A5 (µM)
Compound B2 (µM)
N=3
ADD Excess Inhibition
Chi2=330
Vol=7.18(.098)
Isobologram
Compound A5 / 5.1µM
Compound B2 / .1µM
Additivity
Inhibition=60.0

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2005222163 A
- WO 2010020675 A
- US 5665772 A
- EP 09060793 W


### Non-patent literature cited in the description


- **FISCHER, P. M.** *Curr. Opin. Drug Discovery Dev.,* 2001, vol. 4, 623-634
- **LIU Q. et al.** *Drug Discovery Today: Therapeutic Strategies,* 2009, vol. 6 (2), 47-55
- **GOY A.** *Current Oncology Reports,* 2007, vol. 9 (5), 391-398
- **SHAPIRO.** *Journal of Clinical Oncology,* 2006, vol. 24 (11), 1770-1783
- **FASOLO.** *Expert Opin. Investig. Drugs,* 2008, vol. 17 (11), 1717-1734
- **TALLARIDA RJ.** An overview of drug combination analysis with isobolograms. *Journal of Pharmacology and Experimental Therapeutics,* 2006, vol. 319 (1), 1-7
- **LEHAR et al.** Synergistic drug combinations improve therapeutic selectivity. *Nat. Biotechnol.,* July 2009, vol. 27 (7), 659-666